# EUROPEAN PATENT APPLICATION

(11) **EP 4 306 651 A1**
(43) Date of publication of application: **17.01.2024**
(21) Application number: 23184416.8
(22) Date of filing: 10.07.2023
(51) Int. Cl.: C12Q 1/6806

(54) **COMPOSITION AND THE USE OF CELL LYSIS REAGENTS**

(30) Priority: 10.07.2022 US 202263359854 P
(71) Applicant: Vilnius University, 01513 Vilnius (LT)
(72) Inventor: Mazutis, Linas, Vilnius (LT); Goda, Karolis, Vilnius (LT)
(74) Representative: Petniunaite, Jurga

(57) **Abstract**

The present invention relates to amphiphiles and specifically the amphiphiles that can be used as cell lysis reagents. The invention reveals the amphiphiles as well as their combinations for efficiently lysing the mammalian cells under relatively mild conditions that are compatible with a reverse transcription and polymerase chain reaction. The invention reveals the use of mixture of amphiphiles to improve the cell lysis, and obtain increased yields of copy DNA during the reverse transcription reaction. The invention also provides a method for increasing the diversity of gene and transcript capture during single-cell RNA-sequencing using droplet microfluidics.

## Description

### TECHNICAL FIELD

The present invention relates to cell lysis reagents for increasing the gene and transcript capture during copy DNA synthesis.

### BACKGROUND OF THE INVENTION

Gene expression analysis constitutes one of the most common techniques in cell and molecular biology. To evaluate the gene expression, the cells must be first disrupted to release their intracellular nucleic acids (e.g., RNA), which can then be captured, amplified and analysed according to the needs of a study. To ensure efficient RNA release, the cells are typically exposed to lysis reagents such as detergents (surfactants), which preferentially bind to cell membrane and solubilize lipids.

In gene expression and transcriptomics studies cell lysis is typically performed under conditions that minimally affect the activity of nucleic acid processing enzymes. Finding the right balance between the efficient cell lysis and compatibility with subsequent enzymatic steps often relies on extensive trial-and-error efforts. So far, the non-ionic detergents belonging to the TRITON, TWEEN and IGEPAL class have been used most extensively in cell and molecular biology assays, where cell lysis and nucleic acid analysis are conducted without a nucleic acid purification step. However, the "lysis" efficiency of non-ionic detergents for releasing the intracellular nucleic acids for subsequent analysis, remains poorly characterized. The large variety of chemical structures and properties makes it difficult to establish the general rules for the efficiency of cell lysis and nucleic acid release [1]. Typically, ionic detergents provide more efficient means for cell membrane solubilization as compared to the non-ionic or zwitterionic ones [2], yet the use of ionic detergents (e.g., SDS) is undesirable because they tend to inhibit enzymatic reactions (e.g., by denaturation of proteins). In situations where a given enzymatic reaction and cell lysis are performed in the same reaction mix, the use of enzyme-compatible cell lysis reagents is required. Relevant to this invention, for the enzymatic reactions involving reverse transcription (RT) reaction, polymerase chain reaction (PCR), and nucleic acid amplification and synthesis, it is desirable to use non-inhibitory lysis reagents that can efficiently disrupt the cells and release intracellular nucleic acids. Such non-inhibitory lysis reagents (detergents) often belong to the TRITONS, TERGITOLS, TWEENS and BRIJ class. It has been shown that in many cases the optimal hydrophobic chain of non-ionic detergents is ~18 and that hydrophilic head length can effect cell lysis efficiency [3]. The size of the surfactant head group can be a determining factor for the efficacy by which a surfactant is able to disrupt a cell membrane [4]. Many non-ionic surfactants show fairly high (3 × 10³/M) membrane partition coefficient. For example, Triton X-100 can be regarded as a "strong" detergent because the large headgroups affect the lipid packing and may lead to a more efficient lipid membrane disruption. In contrast, "weak" detergents typically have small headgroups. The tritons, CmEOn with n = 7 to 8, and the alkyl maltosides are "strong" detergents, whereas the alkyl glucosides and C12EOn with n = 3- 6 are "weak" detergents [5].

The extent of the surface activity of a detergent is mainly determined by the hydrophilic-hydrophobic balance of the molecule [6]. At concentrations just below the critical micelle concentration (CMC) individual detergent molecules or small aggregates causes the membrane to become more permeable. When the bilayer becomes saturated with detergent, detergent-lipid micelles may appear in the aqueous medium, and membrane solubilization will take place. Usually mixed micelles occur only at or near the CMC of the pure detergent. At detergent concentrations close to or above the CMC range, rapid breakdown of the membrane typically occurs [7]. Loss of cell viability is typically recorded below CMC [8], before full membrane solubilization.

When detergent is applied to membranes, solubilization typically occurs by three successive stages: detergent incorporation, lamellar-micellar phase transition (formation of mixed micelles) and total solubilization (separation of protein and lipid mixed micelles). The insoluble fraction that remains after progression through these events is typically enriched in sphingolipids, cholesterol and raft proteins [9]. Even though increasing the temperature increases the lipid solubilization, up to 30% of lipids may remain insoluble under these change in environment [10]. As a result, cell lysis and thus RNA release using only non-ionic detergent might be limited because it may generate detergent resistant cell membranes and prevent efficient release of mRNA molecules or restrict RT enzyme access to some mRNA molecules.

The use of cholesterol-binding detergents and compounds often facilitates membrane solubilization by non-ionic detergents [11-13]. In particular, cholesterol-binding molecules tend to disrupt protein-lipid interactions [11], facilitate protein solubilization by non-ionic detergent [10, 14, 15] and by doing so improve the overall cell lysis efficiency. However, whether the use of cholesterol-binding detergents (e.g., saponin, methyl-β-cyclodextrin, digitonin, etc.) is compatible with reverse transcription (RT) reaction and whether their use alone or in combination with non-ionic detergents improves copy DNA (cDNA) yields, remains so far unknown and uncharacterized.

Herein, we reveal that the use of cholesterol-binding compounds may improve cDNA yields when used alone or in combination with non-ionic detergents. Specifically, we reveal that the use of triterpene glycosides (also known as saponins) alone, or in combination with non-ionic detergents, is compatible with single-cell RNA-Seq protocols, does not destabilize water-in-oil emulsions at elevated temperatures (>37 ºC), and increases the diversity of RNA species captured from lysed-cells during the reverse transcription reaction.

Many state-of-the-art molecular biology protocols for single-cell nucleic acid analysis rely on a step where single-cells, or a mixture of cells, are lysed to release their intracellular nucleic acids [16-30]. Some lysis reagents (e.g., SDS, guanidinium isothiocyanate), despite being highly efficient in lysing the cells, cannot be employed because their presence in the cell lysate may inhibit subsequent enzymatic reactions. For example, it is well known for a person experienced in the field that even small traces of SDS (<0.01%) may inhibit the reverse transcription and/or PCR reactions [31]. Therefore, for many molecular biology protocols that are based on nucleic acid analysis it is highly desirable to employ such cell lysis reagents that would efficiently lyse the cells to release their intracellular RNAs, and would not inhibit subsequent RT and/or PCR reactions. In addition, it is highly desirable to employ such lysis reagents that would improve intracellular RNA release and/or increase RNA capture and/or cDNA yields. As we reveal in this invention, the use of triterpene glycosides and in particular the use of saponin for cell lysis, solves the unmet need by improving intracellular RNA release upon cell lysis and increasing cDNA yields. Moreover, the disclosed composition improves the transcript capture of individual cells when performing single cell RNA-Seq experiments. The cell lysis reagents disclosed in this invention may be used directly in the RT, RT-PCR or PCR reactions. As a result, the use of triterpene glycosides with or without non-ionic detergents may improve analytical sensitivity of a large variety of assays that rely on efficient single-cell lysis, and subsequent nucleic acid amplification and analysis. Experienced in the art will be able to adapt the disclosed lysis reagents and composition for different molecular biology protocols involving single-cells, or mixture of cells. The non-limiting examples of assays that may benefit from the disclosed invention include RT, PCR, RT-PCR, qPCR, RT-qPCR sequencing and a large variety of single-cell -omic assays [16-30] amongst other. Finally, another important aspect of the invention relates to the fact that the use of triterpene glycosides and in particular the use of saponin, with or without non-ionic detergents, does not compromise the emulsion stability during elevated temperatures (> 37 ºC), when the water droplets are suspended in the fluorinated carrier oil comprising fluorosurfactant.

### SUMMARY OF THE INVENTION

According the present invention, there is provided a composition of cell lysis reagent comprising a triterpene glycoside amphiphile that preferentially binds to fatty acids, phospholipids, glycolipids, and/or sterols and provided that said triterpene glycoside amphiphile does not lower the activity of the enzymatic reaction needed to synthesize DNA by more than 10%. The invention further provides that the cell lysis reagent does not inhibit the copy DNA synthesis by the reverse transcriptase. In one aspect, the triterpene glycoside is saponin.

In a second aspect, the composition further comprising triterpene glycoside amphiphile used in combination with the non-ionic amphiphile belonging to the class of detergents comprising of n-alkyl-glucopyranosides, n-alkyl-maltosides, n-alkyl-thioglucopyranosides, ethoxylated nonylphenols (known as different types of Tergitol), octylphenoxypolyethoxy ethanols (known as different types of Triton and Igepals), polyoxyethylene ethers (known as different types of Brij) and alkyl polyethylene glycol ethers, genapols.

In a third aspect, the triterpene glycoside amphiphile belongs to the class of detergents that bind the cholesterol such as CHAPSO/CHAPS, Digitonin, DC-cholesterol, Cholesteryl-PEG600, CHOBIMALT, CHAPSTEROL, Saponin, or cyclodextrin. Further, the non-ionic amphiphile(s) is one of, TERGITOL 15-S-7, TERGITOL 15-S-9, TERGITOL 15-S-30, TERGITOL 15-S-40, TERGITOL NP7, NP40, TRITON X100, GENAPOL C100, IGEPAL CA-630, IGEPAL CA-720, BRIJ58, BRIJ35, and TWEEN20.

In a fourth aspect, the concentration of the amphiphile(s) is in the range of 0.00001 % to 10% (w/v), but more preferably in the range of 0.001 % to 1% (w/v).

In a fifth aspect, according to the present invention, it is provides a composition of a reaction mixture that comprises cell lysis reagents, triterpene glycoside and non-ionic amphiphiles, that preferentially bind to fatty acids, phospholipids, glycolipids, and/or sterols and that do not inhibit the enzymatic reaction needed to synthesize DNA, and further provided that the reagent concentrations are 0.001 % to 1% (w/v) and reagents necessary for DNA synthesis, and further that cell lysis and DNA synthesis reactions are performed in the same reaction mix.

In a sixth aspect, the present invention provides a method of cell lysis and DNA synthesis, the method comprising adding a cell(s), cell lysis reagents, comprising triterpene glycoside with or without a non-ionic amphiphile, and DNA synthesis reagents to a reaction mixture; lysing the cell(s) to release the desired component from the cell, wherein the desired component is nucleic acid; and amplifying the nucleic acid.

In a seventh aspect, DNA synthesis reagents comprises reverse transcription (RT) reagents in the method.

In an eighth aspect, DNA synthesis reagents comprises PCR reagents in the method.

In a ninth aspect, the reaction mixture of the method is loaded in a microreactor such as micro-well, nano-well, water-in-oil droplet, or any other compartment that has volume below 10 µl and above 1 pl.

In a tenth aspect, the reaction mixture of the method is loaded on a microfluidic device or other device, assembly or instrument capable of forming a water-in-oil droplet.

In an eleventh aspect, the present invention provides a method of cell lysis and DNA synthesis on single-cells comprising adding cells, DNA barcodes, cell lysis reagents comprising triterpene glycoside with or without of non-ionic amphiphiles, and DNA synthesis reagents in to water-in-oil droplets suspended in the fluorinated carrier oil, further comprising a fluorosurfactant; lysing the cell(s) to release the desired component from the cell, wherein the desired component is nucleic acid; and amplifying the nucleic acid.

In yet another aspect, the method provides encapsulating a plurality of cells and a plurality of DNA barcoding beads in a plurality of droplets together with the reaction composition, wherein each bead of the plurality of beads comprises a molecular tag, and some droplets comprise cells and beads, and further provided that the plurality of droplets comprises cell lysis reagents, RT reaction reagents, and DNA synthesis reagents and no more than one cell and no more than one bead, and the molecular tag of the bead in one droplet is distinguishable from the molecular tags of the beads in the other droplets; lysing the cells; and converting RNA into cDNA using molecular tags at RT primers.

In a further aspect of the method, it is provided that the molecular tag is released from the bead and the molecular tag is attached to the nucleic acid in the droplet released by lysed cell. The molecular tag is a barcoding DNA oligonucleotide, which allows the nucleic acid in one droplet to be uniquely identified from among the nucleic acids of the other droplets.

In aspects relating to the provided methods, it is provided that nucleic acid amplification is chosen from one of: polymerase chain reaction (PCR), loop-mediated isothermal amplification (LAMP), isothermal amplification, linear amplification, reverse transcription, replication, polymerase chain reaction (PCR), quantitative PCR, real-time PCR, digital PCR, reverse transcriptase PCR (RT-PCR), multiplex RT-PCR, and any combination thereof.

In a final aspect, the present invention provides a kit for cell lysis comprising a triterpene glycoside, a kit for cell lysis comprising triterpene glycoside and a non-ionic amphiphile, and a kit for DNA synthesis reaction comprising a reaction composition for cell lysis and DNA synthesis.

### BRIEF DESCRIPTION OF THE DRAWINGS

To assist understanding of the present disclosure and to show how embodiments may be put into effect, reference is made by way of example to the accompanying drawings described below. It should be noted that in the schematics provided various aspects are not drawn to scale.

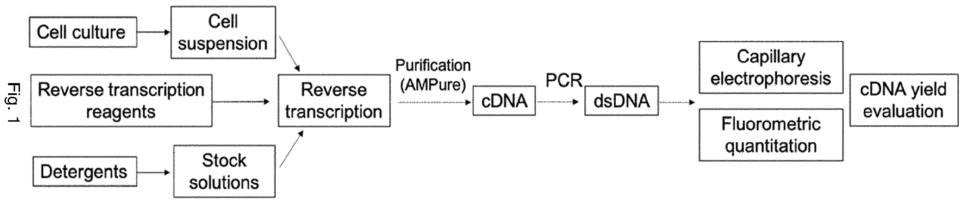

Fig. 1. Shows schematics of cDNA yield evaluation under different lysis conditions. Even number of cells is loaded into a tube together with lysis reagents and RT reaction mix. Next, the reaction is incubated at conditions sufficient for cDNA synthesis to occur. The resulting cDNA is purified, PCR-amplified and analysed using capillary electrophoresis and fluorometer. Finally, the yield of cDNA is compared along different lysis conditions.

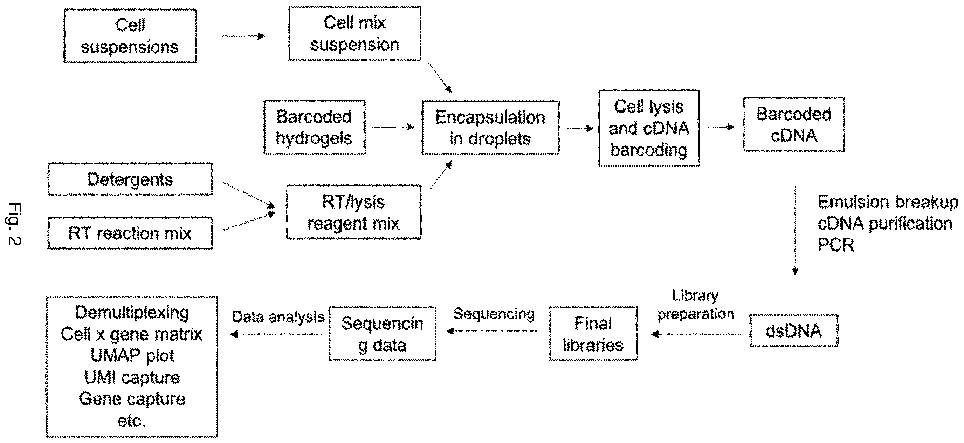

Fig. 2. Shows schematics of single-cell RNA-Seq experiment using different lysis conditions. Different cell lysis reagents were loaded in droplets along with following the protocol published previously (Zilionis et al., Nature Protocols, 2017). The encapsulated cells were lysed, cDNA barcoded, and the capture of mRNA and genes was evaluated by next-generation sequencing.

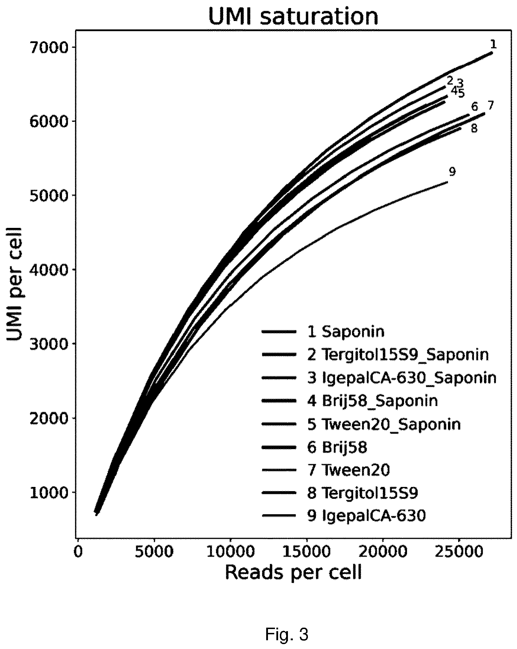

Fig. 3. Shows transcript (UMI) capture in single-cells under different lysis conditions. Numbers represent different lysis conditions. Using a saponin alone or in a mixture with non-ionic detergents improves transcript capture than using the same non-ionic detergents alone.

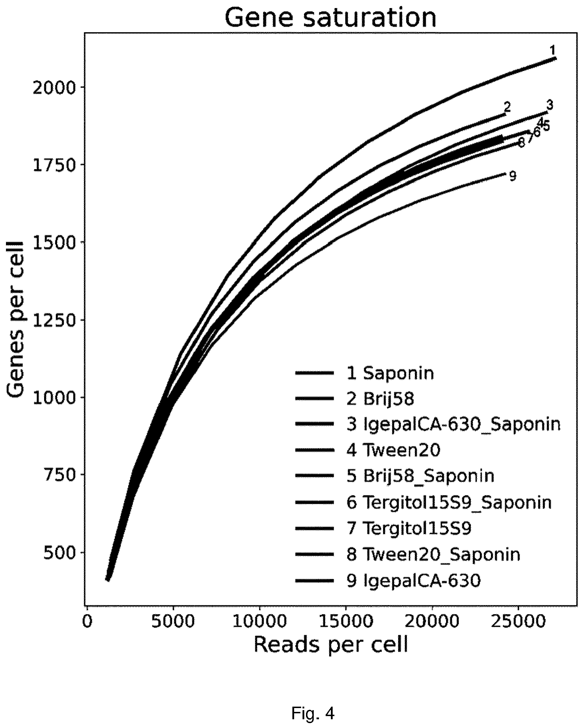

Fig. 4. Shows gene capture in single-cells under different lysis conditions. Numbers represent different lysis conditions. Using a saponin alone or in a mixture with non-ionic detergents improves gene capture than using the same non-ionic detergents alone.

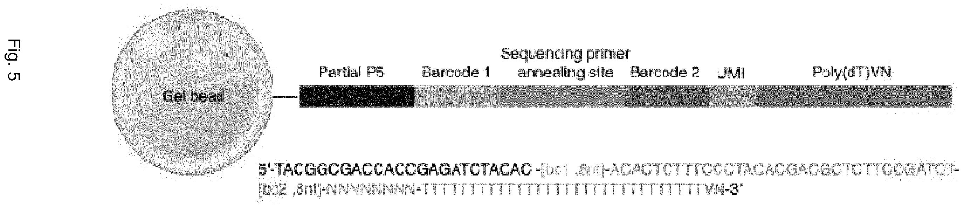

Fig. 5. Shows schematics of barcoded hydrogel bead. Vertical boxes indicate different section of the oligonucleotide attached to the hydrogel bead. The nucleotide sequence is also provided.

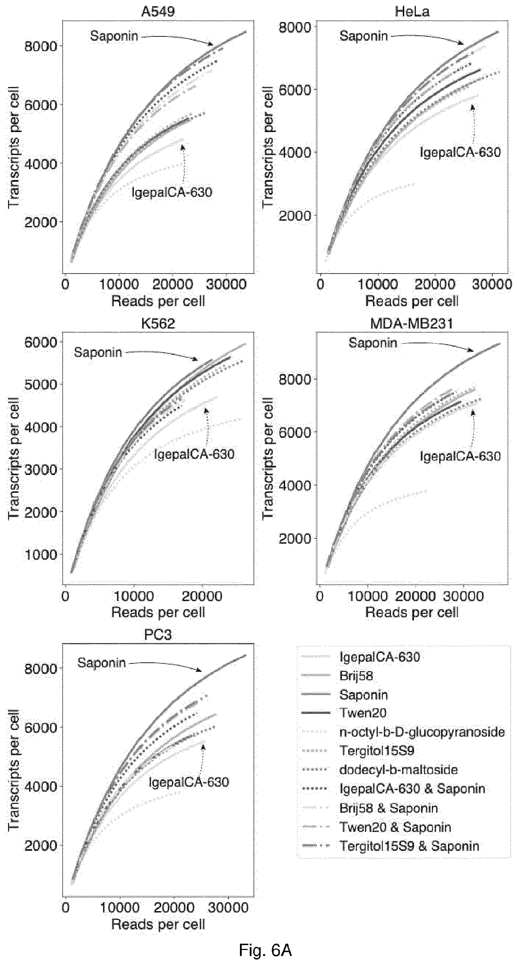

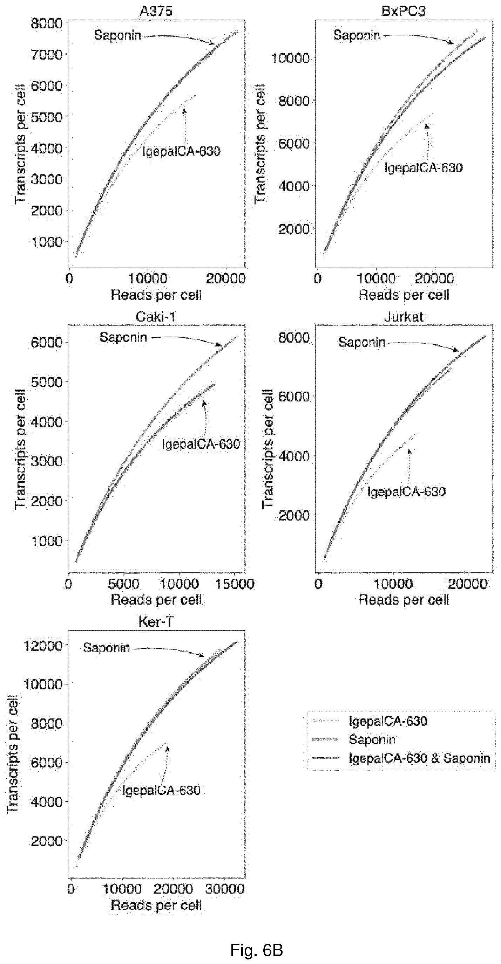

Fig. 6. Shows transcript (UMI) capture in single cell transcriptomics study under different lysis conditions. The plot presents transcript count (Y-axis) in single-cells at a given sequencing depth (X-axis). Using triterpene glycosides (e.g. saponin) alone or in a mixture with non-ionic detergents improves transcript recovery (detection) as compared to other non-ionic detergents. A) Transcript (UMI) detection in K562, A549, HeLa, MDA-MB231, PC3 cell lines. B) Transcript (UMI) detection in A375, BxPC3, Caki-1, Jurkat and Ker-Tomato cells.

### DETAILED DESCRIPTION

Throughout the text the terms of 'comprising' and 'containing' have been used interchangeably and have the same meaning.

Articles such as "a," "an," and "the" include the singular and the plural reference unless the context clearly indicates otherwise. For example, a reference to "a cell" includes one cell and two or more of such cells.

The terms "detergent", "surfactant", "amphiphile" are used herein interchangeably and have the same meaning.

In the context of this inventions, if the presence of amphiphile in the reaction mix reduces the catalytic activity of DNA polymerase by less than 10%, it is shall be considered as non-inhibitory (absence of inhibition).

The present invention generally relates to the composition and methods of lysing cells for the purposed of nucleic acid processing and analysis. In particular, in one aspect it relates to a method of lysing a mixture of cells or single-cells for the purpose of converting the cellular RNA into cDNA in the presence of lysis reagents. Certain aspects are generally directed to methods for performing reverse transcription (RT) and/or polymerase chain reaction (PCR) in the presence of cell lysis reagents.

The methods of the present invention may also comprise lysing the single-cell, or more than one cell, in the presence of nucleic acid processing enzymes, and that upon the cell lysis the nucleic acid processing enzyme retains most of its catalytic activity. In some scenarios, the presence of lysis reagents in the reaction mix may provide stabilizing effect, meaning that enzyme will retain catalytic activity under given conditions for a longer period of time as compared to the conditions where the lysis reagents are absent.

The present invention also relates to the composition and methods of lysing cells under conditions that do not substantially inhibit reverse transcription reaction so that RNA molecules released from lysed cells can be captured and converted to cDNA by an RT enzyme, in the presence of lysis reagents.

The disclosed invention may be relevant to a large variety of methods and techniques that involve processing of nucleic acids from lysed cells. Efficient cell lysis is particularly important in assays that aim to evaluate the transcriptional activity of single-cells [16-30]. For example, assays that are used to evaluate the gene expression of a cell, require a lysis step in order to release the intracellular nucleic acid molecules and make them accessible to biochemical reactions. Similarly, the assays that are used to evaluate RNA content of cells or tissues, require a lysis step in order to release the RNA molecules encoded by the said cells and make them accessible to biochemical reactions.

The present invention also relates to certain amphiphiles and their combination thereof to improve cell lysis and increase the diversity of RNA species that may be captured and amplified by RT and PCR. In particular, the use of triterpene glycosides and more specifically the use of saponins may improve cell lysis efficiency and as a result ensure better RNA capture as compared to the commonly used lysis reagents in the state-of-the-art methods. In some scenario the use of saponin in combination with non-ionic detergent(s) may provide higher cDNA yields as compared to reaction conditions where the same non-ionic detergent or saponin are used alone.

Using the amphiphiles disclosed in this invention more than 50% of the total RNA must be released from the lysed mammalian cell.

Using the amphiphiles disclosed in this invention more than 5% of all mRNA molecules must be captured from the lysed mammalian cell during RT reaction.

In particular, the present invention involves lysis reagents belonging to triterpene glycosides, ethoxylated nonylphenols (known as different types of Tergitol non-ionic detergents), octylphenoxypolyethoxy ethanols (known as different types of Triton and Igepal non-ionic detergents), polyoxyethylene ethers (known as different types of Brij non-ionic detergents) and alkyl polyethylene glycol ethers, or any combination thereof, to achieve efficient cell lysis and obtain increased cDNA yields upon RT reaction. Moreover, said lysis reagents or combination thereof may be used to achieve a better capture of transcripts in single-cells.

Because the biological entities, such as cells, are efficiently lysed in the presence of triterpene glycosides, their intracellular RNA is released and becomes readily accessible to nucleic acid processing enzymes. Therefore, using the triterpene glycoside alone or in mixture with non-ionic detergents, one can release more RNA from lysed cells and thus obtain higher yields of cDNA and capture higher diversity of RNA molecules.

The present invention also simplifies the use of laboratory protocols that involve RNA capture and cDNA synthesis. Because disclosed lysis reagents are compatible with nucleic acid processing enzymes (e.g., RNA polymerase, DNA polymerase, RT enzyme), the cell lysis and nucleic acid analysis (e.g., reverse transcription) can be performed in the same reaction mix, without a need to purify the nucleic acids. This simplifies the use of laboratory protocols that involve RNA capture and cDNA synthesis.

Another important feature of the invention is that the disclosed composition of cell lysis reagents does not destabilize emulsion droplets at elevated temperatures. Therefore, a plurality of single-cells isolated in a plurality of microfluidic droplets can be efficiently lysed and their RNA converted to cDNA without a negative effect on droplet stability. As a result, the composition of the cell lysis reagents described herein have broad applicability in high-throughput analytical methods where efficient cell lysis is required under conditions compatible with reverse transcriptase enzymes.

The disclosed composition of cell lysis reagents in combination with reagents necessary for DNA synthesis can be used for a method of cell lysis and DNA synthesis in the same reaction mix, as shown in Fig. 1. The cells, lysis reagents and reverse transcription reagents are mixed together to create one reaction mix. The mRNA released from the lysed cells is reverse transcribed using oligonucleotides into a copy DNA (cDNA). The resulting cDNA is then purified using AMPure purification kit, PCR-amplified and analysed by capillary electrophoresis (BioAnalyzer). The DNA amount is also evaluated by fluorometer (Qubit) and the cDNA yields between different lysis conditions is compared to the reference (0.1% NP40).

The present invention is also directed to a method comprising a step of encapsulating cells, lysis reagents, RT reaction mix and barcoding beads in microfluidic droplets. In particular, the present invention involves lysis reagents that do not compromise water-in-oil droplet stability during cell encapsulation, cell lysis, reverse transcription reaction and incubation at high temperatures (>80 ºC) for extended periods of time (>10 min). As detailed below the droplets are made on a microfluidics device using fluorinated liquid as a carrier oil and which is supplemented with a fluorinated surfactant.

The methods of the present invention may also comprise a step of encapsulating the cells. In particular, it may comprise a step of encapsulating a plurality of cells into a plurality of microfluidic droplets with lysis reagents. Those of ordinary skill in the art will be aware of techniques for encapsulating cells within microfluidic droplets [32, 33]; see also for example, U.S. Pat. Nos. 7708949, 8337778, 8765485 each incorporated herein by reference. The cells preferably are encapsulated at a density such that on average one droplet would contain one cell or less. In a non-limiting example, the lysis reagents are encapsulated with cells and hydrogel beads carrying DNA barcodes.

In one set of embodiments, the cells are loaded in microfluidic droplets such that, on average, each droplet has less than 1 cell in it. For example, the average loading rate may be less than about 1 cell/droplet, less than about 0.8 cells/ droplet, less than about 0.6 cells/ droplet, less than about 0.4 cells/ droplet, less than about 0.3 cells/ droplet, less than about 0.2 cells/ droplet, less than about 0.2 cells/ droplet, less than about 0.1 cells/ droplet, less than about 0.05 cells/ droplet, less than about 0.01 cells/ droplet.

The present invention makes use of such droplets to lyse encapsulated cells in order to release the nucleic acids from the said cells, and so that the nucleic acids can be processed further using DNA polymerization reaction.

In some embodiments the methods of the invention use droplets comprising cells and lysis reagents to disrupt the cell membrane and release nucleic acids. In other embodiments, the methods may comprise the steps of lysing the cells by increasing the temperature in the presence of lysis reagents.

In some cases, the nucleic acids that are released upon exposure to cell lysis reagents of this invention may be subjected to further processing, for example, by including suitable reagents specific to the nucleic acid processing method. Examples of nucleic processing known to those of ordinary skill in the art include, but are not limited to, reverse transcriptase, primer extension reaction, polymerase chain reaction, multiple displacement amplification, poly(A) tailing, nucleic acid hydrolysis, ligation to an oligonucleotide, modification of 5'- or 3'- end, or both. As in one non-limiting example of the invention that is shown in Fig. 2, the cells are isolated in microfluidic droplets, lysed with lysis reagents to release mRNA and reverse transcribed in the droplet using barcoding DNA oligonucleotides. The resulting barcoded cDNA is released from droplets, purified using AMPure kit, PCR-amplified and processed through library construction as described in details in Example 2.

Also provided by the present invention is a kit for improved lysis of the cells and release of intracellular nucleic acids, comprising amphiphiles at a concentration between 0.01-50% (w/v).

The kit may comprise triterpene glycoside (e.g., saponin). The kit may comprise non-ionic detergents such as ethoxylated nonylphenols (commonly known as different types of Tergitols), octylphenoxypolyethoxy ethanols (commonly known as different types of Tritons and Igepals), polyoxyethylene ethers (commonly known as different types of Brijs) and alkyl polyethylene glycol ethers, at concentration 0.01-50% (w/v). The kit may comprise a mixture of triterpene glycoside and non-ionic detergent(s) and can be supplied in a separate container (tube) or supplemented in the reaction mix. For example, a mixture comprising triterpene glycoside and non-ionic detergent(s) can be supplemented in RT reaction buffer. The said lysis reagents can be part of a kit that is used to achieve increased cDNA yields, and/or to achieve a better capture of transcripts from individual cells. The kit may optionally comprise other reagents such as reverse transcription enzyme, buffer, oligonucleotides, dNTPs, microfluidic chip and/or microfluidics consumables (e.g., tubing, needles, etc.). The kit may further comprise instructions for performance of the methods described herein. Finally, the kit and compositions can be adapted to single-cell RNA-Seq assays on hundreds of thousands of isolated cells and molecular species, in a massively parallel fashion.

As discussed below the cell lysis may be performed within water-in-oil droplet. However, it should be understood that other compartments may be used to perform cell lysis. Those experienced in the field will be able to choose the desirable reaction format and volume for conducting cell lysis. For example, single-cells, or a mixture of cells, can be lysed in the laboratory tubes, well(s) of a microtiter plate, micro- or nano-wells, in microreactors, cages or other compartments that have reaction volumes smaller than 1 millilitre but larger than 1 picolitre (pl), and more preferably cell lysis is performed in the compartments that have volumes larger than 1 pl and smaller than 100 µl and even more preferably having volumes larger than 10 pl and smaller than 1 µl.

The barcoded hydrogel (bead) that is co-encapsulated into the droplet with a cell, lysis reagents and reaction mix may comprise a reagent for use in the processing of the nucleic acid, for example a molecular tag or label, such as a nucleic acid tag or a barcode, an enzyme, an antibody, a lytic reagent, a DNA primer, or a dye. In a preferred embodiment of the methods of the invention the bead comprises an oligonucleotide comprising a barcode.

In particular, a barcode is a user-defined DNA sequence preferably longer than 4 nucleotides but shorter than 100 nucleotides and more preferably in the range of 6-70 nucleotides and even more preferably in the range of 8-16 nucleotides long.

In the methods of the invention discussed herein, where nucleic acid from a plurality of cells is being barcoded, the diversity of unique barcode sequences is at least 100, and more preferably more than 1000, and more preferably more than 10,000 and more preferably more than 100,000 and even more preferably more than 1,000,000 but less than 10^12.

In a preferred embodiment the barcode is comprised in a barcoding DNA oligonucleotide which is 12 to 300 nucleotides in length, preferably 20 to 150 nucleotides in length, and more preferably 30 to 120 nucleotides in length. The barcoding DNA oligonucleotide may further comprise one or more of (i) a unique molecular identifier (UMI), wherein the UMI is a random nucleotide sequence longer than 4 nucleotides but shorter than 50 nucleotides, and preferably in the range of 4-12 nucleotides and still more preferably 8 to 16 nucleotides in length; (ii) a cell barcode preferably longer than 4 but shorter than 100 nucleotides, and more preferably 6 to 70 nucleotides in length, (iii) a sequence able to specifically bind to a region of interest in the nucleic acid (e.g. a poly-T sequence or a gene specific sequence); (iv) an overhang, (e.g. a sticky end) or a blunt end; and (v) an adapter sequence (e.g. a PCR adapter and/or a sequencing adapter and/or hybridization adapter).

The beads used for co-encapsulation may be solid particles comprising a molecular tag [34] or squishy particles (hydrogel beads) comprising a molecular tag [17]. The bead may be hard or soft, it can be made of organic or inorganic material, it can be a solid particle, a hydrogel particle, a hydrogel bead, or composite hydrogel bead. It may also comprise polyacrylamide, agarose, polystyrene and/or poly-N-isopropylacrylamide. Preferably the particle has a size of in the range of 1-100 µm and preferably in the range of 10-80 µm, and more preferably in the range of 20-70 µm, and more preferably approximately 60 µm.

It is preferred that the reagent, and in particular the molecular tag or barcoding DNA oligonucleotide referred to herein, is covalently attached to the bead. However, this is not essential and in other examples, the bead may comprise a short single stranded nucleotide stub to which the reagent base-pairs.

Where the attachment is covalent, it may comprise a cleavable linker, such as a photocleavable linkers, a chemically cleavable linker, or an enzymatically cleavable linker, such that the reagent and in particular the molecular tag or barcoding DNA oligonucleotide referred to herein can be released from the bead by exposing the plurality of droplets to illumination (preferably below 450 nm wavelength), to a chemical agent (e.g. a reducing agent), or an enzyme (e.g. endonuclease), respectively, to release the molecular tag covalently attached to the bead when the bead is in the droplet.

However, it should be noted that the methods described herein do not always involve a step of releasing the reagent (e.g. the molecular tag or barcoding DNA oligonucleotide) from the bead. In particular, the reagent may remain attached to the bead inside the droplet, while the cell is lysed inside the droplet to release the nucleic acids and allow it to come into contact with the reagent. In other embodiments the bead may comprise more than one reagent. In an example of such an embodiment, the bead may comprise two molecular tags, e.g. one barcoding DNA oligonucleotide that is to be attached to DNA released from the lysed cell, and one barcoding DNA oligonucleotide that is to be attached to cDNA produced with a reverse transcriptase reaction with the RNA released from the lysed cell. In such an embodiment it may be desirable for the barcoded DNA to become attached by the barcoding DNA oligonucleotide to the bead, while the barcoded RNA is untethered, or vice versa. The cells are co-encapsulated in a droplet. The droplet may be a microfluidic droplet, i.e. a droplet generated with a microfluidic device. Preferably the droplet is an oil-in-water droplet generated using a microfluidic device.

Such microfluidic droplets have particular utility in high throughput methods involving a large number of samples of biological species and in particular a large number of cells. Accordingly, the method of the present invention can be readily scaled to attach barcodes to a large number of cells and samples.

One non-limiting example of the invention is shown in Fig. 2. A plurality of cells is injected into a microfluidics chip along with plurality of hydrogel beads, lysis and assay reagents. Those of ordinary skill in the art will be aware of techniques for loading hydrogel beads in microfluidic droplets [35, 36] and for co-encapsulating cells within microfluidic droplets with hydrogel beads [17, 18, 36]; see also for example, U.S. Pat. 10596541 incorporated herein by reference. The cells are introduced to a microfluidics chip at such dilution that each microfluidic droplet would preferably contain, on average, no more than one cell. Simultaneously, the hydrogel beads are injected into a microfluidics chip at such flow rates that each microfluidic droplet would preferably contain one bead. Therefore, adjusting the flow rates it is possible to achieve conditions where most of the cells will be co-encapsulated with one bead.

In some embodiments, the encapsulation conditions are chosen such that droplets contain one hydrogel bead. The presence of empty droplets and/or droplets with single hydrogel beads but without cells, and/or droplets with cells but without hydrogel beads, do not substantially affect performance. The co-encapsulated cells and hydrogel beads may be collected in the form of an emulsion and processed according to the aim of the particular application. For example, in one particular embodiment, the RNA of lysed cells is converted into barcoded complimentary DNA upon reverse transcription or other DNA polymerization reaction with barcoded oligonucleotides i.e., introduced with a hydrogel bead.

In one set of embodiments, the nucleic acid molecules may be "barcoded" or include unique sequences attached that can be used to distinguish nucleic acids in a droplet from those in another droplet, or even when the nucleic acids are pooled together. The barcoded oligonucleotides having unique sequences are delivered to individual droplets by hydrogel beads attached thereto and the nucleic acids containing within the droplets are "barcoded" during enzymatic reaction by barcoded oligonucleotides. The barcodes are used to distinguish nucleic acids, e.g., originating from different cells. The barcoded oligonucleotides attached to nucleic acid molecules within a droplet may be distinguishable from oligonucleotide tags in other droplets. Barcoding nucleic acid molecules is important when pooling the nucleic acids from different droplets.

In another set of embodiments, the barcoding DNA oligonucleotide are introduced into the droplets by a hydrogel bead or a solid particle and then released from the bead once the bead is loaded into a droplet. When the hydrogel beads are loaded within the droplets at a density such that one droplet on average contains no more than 1 bead, then once the barcoding DNA oligonucleotide are released from the bead, then most or all of the droplets will contain one unique barcoding DNA oligonucleotide, thus allowing each droplet (and the nucleic acids contained therein) to be uniquely labelled and identified.

It should be understood that the barcoding DNA oligonucleotide introduced into the droplets by a bead (hydrogel bead or a solid particle) may remain attached to the bead once the bead is loaded into a droplet. When the barcoding DNA oligonucleotide remains attached the bead, the nucleic acids (released by lysed cells) will be captured by the said barcoding DNA oligonucleotide attached to the bead.

It should be understood that the barcoding oligonucleotides are initially attached to hydrogel beads in order to facilitate the introduction of only one unique barcoded oligonucleotide type to each droplet. After introducing a hydrogel bead with barcoding oligonucleotides to droplet and releasing them, a droplet will contain a plurality (typically in other range of 10^3 - 10^9) of barcoded oligonucleotides containing the same unique barcode. Therefore, each barcoding oligonucleotide present within a droplet will be distinguishable from the barcoding oligonucleotide present in the other droplets.

To release barcoding oligonucleotides from the hydrogel-bead a light at approximately 400 nm wavelength range may be applied to cleave the photolabile bond and release barcoding oligonucleotides from the hydrogel. However, it should be understood that this is an example only, and that other methods of cleavage or release can also be used, for example by melting the hydrogel bead with reducing agent [18]. In one set of embodiments, agarose particles containing oligonucleotides may be used, and the oligonucleotides may be released by heating the agarose, e.g., until the agarose at least partially liquefies or softens. However, in some other embodiments, cleavage may be nonessential.

In another set of embodiments, the droplets may be broken to release the barcoded nucleic acids and other contents. The barcoded nucleic acids may then be pooled together and since the barcoded nucleic acids molecules are labelled with different barcoded oligonucleotide tags, the nucleic acids from one droplet (i.e., from one cell) can be distinguished from those from other droplets (i.e. from other cell) by the sequencing of barcoded oligonucleotide tags.

In another aspect, the present invention provides systems and methods for the massively parallel barcoding of DNA or RNA from large numbers of cells. This process may rely on the co-encapsulation of cells along with barcoded nucleic acids, or other suitable oligonucleotide tags attached to hydrogel or polymer beads, together with lysis reagents and/or other reagents that may be used for RNA and/or DNA capture, and/or extension and/or amplification.

In one set of embodiments, the nucleic acid contents of each cell may be labelled with a unique barcode and may allow for hundreds, thousands, or millions of cells to be barcoded in a single experiment for the purpose of determining the cell composition in a population or for screening cell populations.

In yet another aspect, the present invention provides systems and methods for the massively parallel capture, barcoding and quantification of nucleic acid molecules from a large number of single cells, for the purpose of profiling cell populations, characterizing their transcriptome, characterizing their genome, or other purposes.

After purification and optional DNA amplification, the base composition and barcode identity of cellular nucleic acids may be determined, for instance, by sequencing. Alternatively, in some embodiments, DNA oligonucleotides introduced with hydrogel beads may serve as primers for amplification of region of interest in the genomic DNA.

In one non-limiting embodiment, the 3' end of a barcoded oligonucleotide is terminated with a poly-T sequence that may be used to capture cellular mRNA. The 3' end of poly-T oligonucleotide may serve as a reverse transcription primer and may be extended to create cDNA. In one set of embodiments, the 3' end of the barcoded primers may terminate with a random DNA sequence that can be used to capture the RNA or DNA of a cell lysate. In another embodiment, the 3' end of the barcoded primers may terminate with a specific DNA sequence, e.g., that can be used to capture DNA or RNA species ("genes") of interest, or to hybridize to a DNA probe that is delivered into the droplets in addition to the hydrogel beads, together with the enzyme reagents. In another embodiment, a hydrogel bead may carry a number of different primers to target several genes of interest. Analytical techniques can be used to analyze, for example, genomes, transcriptomes, epigenomes, single nucleotide polymorphisms, specific gene expression levels, non-coding RNA, etc. However, the invention should not be limited to only these applications.

In some cases, systems and methods revealed here are related to barcoding the specific set of genes (e.g., tens, or hundreds or even thousands of genes) of individual cells with a unique barcode and prepare genetic material of hundreds, thousands, or even hundreds of thousands or more of individual cells in a single experiment. For example, in some applications it may be desirable to analyze a sub-set of genes of interest, for example between tens to hundreds of genes, rather than whole-transcriptome or whole-genome sequencing.

In some cases, systems and methods revealed here are related to barcoding the DNA fragments arising from individual cells with a unique barcode, and prepare barcoded genetic material from hundreds, thousands, or even hundreds of thousands or more of individual cells in a single experiment.

Some embodiments of the invention may be used to quantify protein abundance in single cells in parallel to RNA or DNA, for example, by first treating cells with DNA-tagged antibodies as discussed in references [19, 20]. After sequencing, the data may be split according to the barcodes and provide information about the molecule count, origin of nucleic acids and/or proteins of interest.

The above discussions are non-limiting examples of various embodiments of the present invention. However, other embodiments are also possible. Accordingly, more generally, various aspects of the invention are directed to various systems and methods for barcoding nucleic acids within microfluidic droplet carrying a cell, cell lysis reagents and other assay reagents and a hydrogel bead carrying barcoded oligonucleotides, as discussed below.

In one aspect, the present invention is generally directed to systems and methods for barcoding nucleic acids within a plurality of microfluidic droplets having an average diameter of the droplet of less than 1000 µm, and more preferably having an average diameter of approximately 100 µm.

The barcoding oligonucleotides may be of any suitable length or comprise any suitable number of nucleotides. The oligonucleotide tags may comprise DNA, RNA, and/or other nucleic acids such as PNA, and/or combinations of these and/or other nucleic acids. In some cases, the oligonucleotide tag is single stranded, although it may be double stranded in other cases. For example, the oligonucleotide tag may have a length of at least about 10 nt, at least about 30 nt, at least about 50 nt, at least about 100 nt, at least about 300 nt, at least about 500 nt, at least about 1000 nt, etc. The length of the barcoding oligonucleotide may vary depending on the application and could be either single-stranded or doublestranded.

As described above, the barcoding oligonucleotides may contain a variety of sequences. For example, the oligonucleotides may contain one or more primer sequences, one or more unique or "barcode" sequences, random sequences, degenerative sequences, one or more promoter sequences, one or more spacer sequences, or the like. Other examples include barcoding oligonucleotides may include a poly-A tail, enzyme recognition sequences, or the like. The oligonucleotide tag may also contain, in some embodiments one or more cleavable spacers, e.g., photocleavable linker. The oligonucleotide tag may be attached to a particle chemically (e.g., via a linker) or physically (e.g., without necessarily requiring a linker).

In addition, in one set of embodiments, a plurality of hydrogel beads, for instance, containing barcoding oligonucleotide tags on their surface may be loaded to droplets, e.g., such that, on average, each droplet contains one hydrogel bead, or less in some cases. After being added to the droplet, the barcoding oligonucleotides may be released from the bead, e.g., using light, reducing agents, or other suitable techniques, to allow the oligonucleotides to become present in solution, i.e., within the interior of the droplet. In such fashion, by loading of the hydrogel beads into the droplets in some embodiments, then releasing the oligonucleotides within the droplet, e.g., to interact with nucleotides or other species, such as is discussed herein.

In one set of embodiments, plurality of droplets is formed in a way that majority of individual droplets would contain no more than one cell and a hydrogel bead comprising barcoding oligonucleotides as described above. A wide variety of different techniques for forming droplets are known to those of ordinary skill in the art. For example, a junction of channels may be used to create the droplets. The junction may be, for instance, a T-junction, a Y-junction, a channel-within-a-channel junction (e.g., in a coaxial arrangement, or comprising an inner channel and an outer channel surrounding at least a portion of the inner channel), a cross (or "X") junction, a flow-focusing junction, or any other suitable junction for creating droplets. See, for example, [37], WO 2004/091763, WO 2004/002627, each of which is incorporated herein by reference.

A relatively large number of droplets may be created, e.g., at least about 100, at least about 500, at least about 1,000, at least about 3,000, at least about 5,000, at least about 10,000, at least about 30,000, at least about 50,000, at least about 100,000 droplets, at least about 300,000 droplets, at least about 500,000 droplets, at least about 1,000,000 droplets, etc.

In yet another set of embodiments, the nucleic acids released from the lysed cells may be tagged with the barcoding oligonucleotide and/or amplified using PCR (polymerase chain reaction) or other suitable amplification techniques. In one set of embodiments, nucleic acid amplification may be performed within the droplets. Those of ordinary skill in the art will be aware of suitable PCR techniques and variations, such as assembly PCR or polymerase cycling assembly, which may be used in some embodiments to produce an amplified nucleic acid. In addition, in some cases, suitable primers may be used to initiate polymerization, or other primers known to those of ordinary skill in the art.

In some cases, the droplets may be broken, or otherwise fused. A wide variety of methods for bursting droplets are available to those of ordinary skill in the art, and the exact method chosen is not critical. For example, droplets contained in a carrying fluid may be disrupted using techniques such as mechanical disruption or ultrasound. Droplets may also be disrupted using chemical agents or surfactants, for example, 1H,1H,2H,2H-perfluorooctanol. Once droplets are broken the nucleic acids (labelled with barcoding oligonucleotides) may be pooled or combined together, amplified, sequenced, etc. Because nucleic acids from different droplets are tagged with barcoding oligonucleotides the nucleic acid molecules can be computationally deconvoluted.

In one set of embodiments, the microfluidics chip is manufactured from an elastomeric polymer such as polydimethylsiloxane ("PDMS"), polytetrafluoroethylene ("PTFE" or Teflon^{®}), or the like [38]. Other examples of potentially suitable polymers include, but are not limited to, polyethylene terephthalate (PET), polyacrylate, polymethacrylate, polycarbonate, polystyrene, polyethylene, polypropylene, polyvinylchloride, cyclic olefin copolymer, polytetrafluoroethylene, a fluorinated polymer, a silicone such as polydimethylsiloxane, polyvinylidene chloride, bis-benzocyclobutene, a polyimide, a fluorinated derivative of a polyimide, or the like. The device may also be formed from composite materials, for example, a composite of a polymer and a semiconductor material.

In a non-limiting example, the PDMS polymer is used which is sold under the trademark Sylgard by Dow Chemical Co., Midland, MI, and particularly Sylgard 182, Sylgard 184, and Sylgard 186.

In a still further aspect, the present invention may provide kits for use in performing the methods described herein. The kits may comprise the lysis reagents, including assay reagents, the beads described herein. In addition, the kits may include one or more microfluidic chips for loading the droplets with cells, lysis and assay reagents and hydrogel beads, as well as microfluidic consumables and appropriate reagents. It should be understood that lysis reagents can be supplied with hydrogel beads or assay reagents or separately.

The following are intended as examples only and do not limit the present disclosure.

### Examples

### Example 1: Evaluation of cell lysis efficiency and cDNA yield in bulk.

The detailed composition and reaction conditions are provided below. Briefly, cell lysis and RT reaction were carried out in bulk with MCF7 and K562 cells. Samples containing 0.02%-0.6% of different cell lysis reagent were evaluated. After the RT reaction, cDNA was pass through a column to remove cell debris and gDNA, purified with 1.2X AMPure, eluted in 20 µl water, amplified by 12-cycles of PCR, purified reaction product with 1.2X AMPure and analyzed on BioAnalyzer chip.

### 1. Preparation of detergents:

Different sets of detergents were prepared in DEPC-water. Each set was tested separately. The cDNA yield for each detergent was compared to the cDNA yield obtained with 0.1% NP40 final concentration.

**Table 1. Different types of detergents that were tested.**

| | Detergent |
|---|---|
| 1. | NP40 |
| 2. | Saponin |
| 3. | Digitonin |
| 4. | Tween 20 |
| 5. | Tween 80 |
| 6. | Tween 60 |
| 7. | Triton X-114 |
| 8. | Triton X-100 |
| 9. | Brij-58 |
| 10. | Brij-35 |
| 11. | Brij C10 |
| 12. | Tergitol (NP10) |
| 13. | Tergitol (NP7) |
| 14. | Pluronic F-68 |
| 15. | Pluronic F-127 |
| 16. | n-octyl-β-D-thioglucopyranoside (OTG) |
| 17. | n-octyl-β-D-glucopyranoside (OBG) |
| 18. | CHAPS |
| 19. | N-Laurylsarcosine |
| 20. | Polyethyleneimine |
| 21. | (1-dodecyl) trimethylamonium bromide (1dTAB) |
| 22. | Deoxycholic acid Na salt |
| 23. | PEG 8000 |
| 24. | Pluronic F-68 |
| 25. | Pluronic F-127 |
| 26. | NP9 |
| 27. | Tergitol TMN 10 |
| 28. | Genapol C100 |
| 29. | Nonidet P40 Substitute |
| 30. | Igepal CA-630 (NP40) |
| 31. | Igepal CA-720 |
| 32. | Tergitol 15-S-7 |
| 33. | Tergitol 15-S-9 |
| 34. | Tergitol 15-S-30 |
| 35. | Tergitol 15-S-40 |
| 36. | methyl-β-cyclodextrin |
| 37. | heptakis(2,6-di-O-methyl)-β-cyclodextrin |
| 38. | imipramine |
| 39. | chlorpromazine |
| 40. | oleic acid |
| 41. | cytochalasin B |
| 42. | 3% ethanol |
| 43. | 3% DMSO |
| 44. | 3% DMF |
| 45. | cyclosporin A |
| 46. | Filipin |
| 47. | α-hederin |
| 48. | hederagenin |
| 49. | hederalchalcoside A |
| 50. | sphingosine |
| 51. | dioscin |
| 52. | nystatin |
| 53. | oleanolic acid |

### 2. Preparation of cells

K562 cells were washed 3 times with ice cold PBS + 0.04% BSA, counted, diluted to 2.2 million cells per 1 mL and stored on ice.

### 3. Preparation of RT reaction mix:

For one 1.54X RT mix with total RNA was prepared for 8 reactions.

| **Reagent** | **Amoun t**, **µL** | **Final concentration after diluting to 1X** |
|---|---|---|
| 5X Maxima RT buffer | 48 | 0.75X |
| 10mM dNTP each | 16 | 0,5 mM |
| RT primer | 8 | 2.5 µM |
| TSO primer | 16 | 10 µM |
| RiboLock (40 U/µL) | 10 | 1,4 U/µL |
| Maxima H minus RT (200 U/µL) | 16 | 10 U/µL |
| Water | 93 | |
| Total volume | 208 | |

**Table 2. List of primers and reagents used in the experiment**

| Name of primer | Forward sequence | | Reverse sequence |
|---|---|---|---|
| **RT primers** | | | |
| RT primer | 5’- CTACACGACGCTCTTCCGATCTNNNNNNTTTTTTTTTTTTTTTTTTTTTT TTTTTTTT-3’ | | |
| TSO | 5'-AAGCAGTGGTATCAACGCAGAGTACATrGrGrG-3' | | |

| **cDNA amplification primers** | | | |
|---|---|---|---|
| REV-primer | 5'-AAGCAGTGGTATCAACGCAGAGT-3' | | |
| FWD-primer | 5'-CTACACGACGCTCTTCCGATCT-3' | | |
| Maxima H minus RT (200U/µL) | | EP0751 | |
| dNTP 10mM each | | R0192 | |
| Ribolock (40 U/µL) | | EO0381 | |
| Dream Taq (5 U/µL) | | EP0701 | |
| AMPure magnetic beads | | | |
| BioAnalyzer HS DNA kit | | 5067-4626 | |
| Qubit BR DNA kit | | Q32850 | |

### 4. Setting up RT reaction:

1. To a 0.2 mL PCR tube 26 µL of 1.54X RT mix were added and mixed with 4 µL of 10X detergent solution (Part I to X).
2. To each tube 10 µL of cell suspension was added and mixed by pipetting.
3. The final volume of reaction was 40 uL.
4. RT reaction was carried out at 50 °C for 60 min followed by inactivation at 85 °C for 5 min.
5. After RT reaction samples were immediately processed further by passing them through Zymo column for 1 min at 10000g.
6. The flow through fraction was cleaned up with 1.2X AMPure beads and eluted in 20 µL of water and processed further.

### 5. cDNA amplification:

7. cDNA amplification mix was prepared as follows:

**Table 3: cDNA amplification mix composition for 8 reactions.**

| **Reagent** | **Volume, µL** | **Final concentration** |
|---|---|---|
| 5X KAPA HiFi Buffer | 32 | 1X |
| KAPA dNTP Mix (10 mM each) | 4.8 | 0.3 mM each |
| TSO-RT-primer (100 µM) | 0.8 | 0.5 µM |
| 10X fwd primer (100 µM) | 0.8 | 0.5 µM |
| KAPA HiFi HotStart DNA Polymerase (1 U/µL) | 3.2 | 0.04 U/µL |
| Water | 54.4 | |

8. 12 µL of cDNA amplification mix were mixed with 8 µL of purified cDNA material resulting in a total of 20 µL solution.
9. Each sample was transferred to thermocycler and PCR program was started:

**Table 4: PCR program settings.**

| **Step** | **Temperature** | **Time** |
|---|---|---|
| Initial denaturation | 98°C | 00:03:00 |
| Denaturation | 98°C | 00:00:15 |
| Annealing | 67°C | 00:00:20 |
| Extension | 72°C | 00:01:00 |
| Go to step 2, 11 cycles (12 in total) | | |
| Final extension | 72°C | 00:01:00 |
| Hold | 4°C | Hold |

10. Amplified cDNA was cleaned up with 1.2X AMPure beads and eluted to 20 µL of water.
11. DNA concentration of each sample was measured with Qubit. Samples were frozen only after cDNA amplification if needed).
12. Calculate the dilution factor needed to reach 1 ng/uL for 0.1% NP40 sample, which is used as a reference.
13. Load 1 µL of each sample (diluted as much times as 0.1% NP40 sample) on BioAnalyzer (BioA) HS DNA kit/chip.

### Results

The yield of cDNA using different types of cell lysis reagents or their combinations is reported in Table 5 below. The "Relative lysis efficiency" was estimated by first quantifying the cDNA yield (ng/µl) obtained at a given lysis condition and then dividing the said cDNA yield by the cDNA yield obtained when using 0.1% NP40.

**Table 5. The yield of cDNA using different types of cell lysis reagents or their combinations**

| Part No. | Detergent | Relative lysis efficiency |
|---|---|---|
| | | |
| I | 0 (reference) | 0.34 |
| I | N P40 0.1 % | 1.00 |
| I | NP40 0.2% | 1.01 |
| I | NP40 0.3% | 1.01 |
| I | NP40 0.4% | 0.98 |
| I | NP40 0.6% | 1.18 |
| I | NP40 0.3% + Saponin 0.1% | 1.70 |
| I | NP40 0.3% + Digitonin 0.1% | 1.18 |
| | | |
| II | 0 | 0.27 |
| II | NP40 0.1% | 1.00 |
| II | NP40 0.3% | 1.02 |
| II | Tween 20 0.1% | 0.98 |
| II | Tween 20 0.2% | 0.99 |
| II | Tween 20 0.3% | 0.99 |
| II | Tween 20 0.4% | 0.99 |
| II | Tween 20 0.6% | 1.02 |
| | | |
| III | 0 | 0.27 |
| III | NP40 0.1% | 1.00 |
| III | NP40 0.3% | 0.92 |
| III | Tween 80 0.1% | 0.50 |
| III | Tween 80 0.2% | 0.69 |
| III | Tween 80 0.3% | 0.72 |
| III | Tween 80 0.4% | 0.71 |
| III | Tween 80 0.6% | 0.70 |
| | | |
| IV | 0 | 0.35 |
| IV | NP40 0.1% | 1.00 |
| IV | NP40 0.3% | 1.00 |
| IV | Brij-58 0.1% | 0.65 |
| IV | Brij-58 0.2% | 1.23 |
| IV | Brij-58 0.3% | 1.20 |
| IV | Brij-58 0.4% | 1.20 |
| IV | Brij-58 0.6% | 1.31 |
| | | |
| V | 0 | 0.34 |
| V | NP40 0.1% | 1.00 |
| V | NP40 0.3% | 0.70 |
| V | Tween 20 0.1% | 0.99 |
| V | Tween 80 0.1% | 0.73 |
| V | Tween 60 0.1% | 0.65 |
| V | Triton X-114 0.1% | 0.75 |
| V | Triton X-100 0.1% | 0.78 |
| | | |
| VI | 0 | 0.28 |
| VI | NP40 0.1% | 1.00 |
| VI | NP40 0.3% | 0.99 |
| VI | Tergitol (NP10) 0.1% | 0.72 |
| VI | Tergitol (NP7) 0.1% | 0.92 |
| VI | Pluronic F-68 0.1% | 0.31 |
| VI | Pluronic F-127 0.1% | 0.48 |
| VI | Brij35 0.1% | 0.97 |
| | | |
| VII | 0 | 0.28 |
| VII | NP40 0.1% | 1.00 |
| VII | NP40 0.3% | 0.94 |
| VII | Brij-58 0.1% | 1.19 |
| VII | n-octyl-β-D-thioglucopyranoside (OTG) 0.1% | 0.31 |
| VII | n-octyl-β-D-thioglucopyranoside (OTG) 0.02% | 0.37 |
| VII | n-octyl-β-D-glucopyranoside (OBG) 0.1% | 0.36 |
| VII | n-octyl-β-D-glucopyranoside (OBG) 0.02% | 0.36 |
| | | |
| VIII | 0 | 0.35 |
| VIII | NP40 0.1% | 1.00 |
| VIII | NP40 0.3% | 1.00 |
| VIII | CHAPS 0.1% | 0.43 |
| VIII | CHAPS 0.02% | 0.43 |
| VIII | N-Laurylsarcosine 0.1% | 0.35 |
| VIII | N-Laurylsarcosine 0.02% | 0.41 |
| VIII | Polyethyleneimine 0.1% | 0.30 |
| | | |
| IX | 0 | 0.53 |
| IX | NP40 0.1% | 1.00 |
| IX | NP40 0.3% | 1.09 |
| IX | Saponin 0.1% | 1.94 |
| IX | Saponin 0.02% | 1.01 |
| IX | Digitonin 0.1% | 0.85 |
| IX | Digitonin 0.02% | 0.85 |
| IX | Polyethyleneimine 0.05% | 0.33 |
| | | |
| X | 0 | 0.34 |
| X | NP40 0.1% | 1.00 |
| X | NP40 0.3% | 1.02 |
| X | (1-dodecyl) trimethylamonium bromide (1dTAB) 0.1% | 0.25 |
| X | (1-dodecyl) trimethylamonium bromide (1dTAB) 0.02% | 0.88 |
| X | Deoxycholic acid Na salt 0.1% | 0.24 |
| X | Deoxycholic acid Na salt 0.02% | 0.51 |
| X | NP40 0.3% + 1dTAB 0.1% | 0.22 |
| | | |
| XI | 0 | 0.45 |
| XI | NP40 0.1% | 1.00 |
| XI | NP40 0.3% | 0.98 |
| XI | NP40 0.3% + Tween 20 1% | 0.18 |
| XI | NP40 0.3% + Tween 80 1% | 0.88 |
| XI | NP40 0.3% + N-laurylsarcosine 0.1% | 0.95 |
| XI | NP40 0.3% + OTG 0.1% | 0.25 |
| XI | NP40 0.3% + Deoxycholic acid Na salt 0.1% | 0.47 |
| | | |
| XII | 0 | 0.36 |
| XII | 0.1% NP40 | 1.00 |
| XII | 0.1 % NP40 + 0.1% Saponin | 1.28 |
| XII | 0.3% Brij-58 + 0.1% Saponin | 1.34 |
| XII | 0.1% Brij-35 + 0.1% Saponin | 1.27 |
| XII | 0.1% Brij-35 + 0.1% Saponin | 1.35 |
| XII | 0.1% Tween20 + 0.1% Saponin | 1.15 |
| XII | 0.1% NP40 + 1% PEG 8000 | 0.65 |
| | | |
| | 0 | 0.37 |
| XIII | 0.1% NP40 | 1.00 |
| XIII | Saponin 0.1% | 1.35 |
| XIII | Saponin 0.2% | 1.34 |
| XIII | Saponin 0.4% | 1.18 |
| XIII | 0.1% NP40 + 0.1% Tween 20 | 0.90 |
| XIII | 0.1% NP7 + 0.1% Tween 20 | 1.01 |
| XIII | 0.1% NP40 + 4.5% PEG 8000 | 0.80 |
| | | |
| | | |
| XIV | 0 | 0.35 |
| XIV | 0.1% NP40 | 1.00 |
| XIV | 0.1% NP40 + 0.1% Polyethyleneimine | 0.34 |
| XIV | 0.1% NP40 + 0.05% Polyethyleneimine | 0.30 |
| XIV | 0.1% NP40 + 0.01% IdTAB | 0.70 |
| XIV | 0.1% NP40 + 0.01% Deoxycholic acid | 0.74 |
| XIV | 0.1% NP40 + 0.1% Pluronic F-68 | 0.66 |
| XIV | 0.1% NP40 + 0.1% Pluronic F-127 | 0.75 |
| | | |
| XV | 0.1% NP40 | 1.00 |
| XV | 0.1% Saponin | 1.64 |
| XV | 0.2% Saponin | 1.84 |
| XV | 0.1 % NP40 + 0.1% Saponin | 1.72 |
| XV | 0.1 % NP7 + 0.1% Saponin | 1.87 |
| XV | 0.1% Brij-35 + 0.1% Saponin | 1.76 |
| XV | 0.1% Tween20 + 0.1% Saponin | 1.68 |
| XV | 0.1% NP40 + 0.2% Saponin | 1.99 |
| XV | 0.1% NP7 + 0.2% Saponin | 2.21 |
| XV | 0.1% Brij-35 + 0.2% Saponin | 1.96 |
| XV | 0.1% Tween20 + 0.2% Saponin | 1.48 |
| | | |
| XVI | 0 | 0.33 |
| XVI | 0.1% NP40 | 1.00 |
| XVI | 0.2% Saponin | 0.97 |
| XVI | 0.1% NP9 | 0.98 |
| XVI | 0.1% Tergitol TMN 10 | 0.83 |
| XVI | 0.1% Genapol C100 | 0.83 |
| XVI | 0.1% Brij C10 | 0.90 |
| XVI | 0.1% NP10 + 0.2% Saponin | 0.94 |
| | | |
| XVII | 0% control | 0.37 |
| XVII | 0.1% NP40 (Igepal C-630A) | 1.00 |
| XVII | 0.1% Brij 58 | 1.03 |
| XVII | 0.1% Brij 58 0.1% Saponin | 1.25 |
| XVII | 0.1% Brij 58 0.2% Saponin | 1.09 |
| XVII | 0.1% Brij 35 | 1.01 |
| XVII | 0.1% Brij 35 0.1% Saponin | 1.19 |
| XVII | 0.1% Brij 35 0.2% Saponin | 1.02 |
| XVII | 0.1% Tergitol NP7 | 0.76 |
| XVII | 0.1% Tergitol NP7 0.1% Saponin | 0.88 |
| XVII | 0.1% Tergitol NP7 0.2% Saponin | 0.92 |
| | | |
| XVIII | 0% control | 0.25 |
| XVIII | 0.1% NP40 (Igepal C-630A) | 1.00 |
| XVIII | 0.1% Genapol C100 | 1.02 |
| XVIII | 0.1% Genapol C100 0.1% Saponin | 1.37 |
| XVIII | 0.1% Genapol C100 0.2% Saponin | 1.21 |
| XVIII | 0.1% Tergitol NP40 | 0.63 |
| XVIII | 0.1% Tergitol NP40 (sigma) 0.1% Saponin | 1.16 |
| XVIII | 0.1% Tergitol NP40 (sigma) 0.2% Saponin | 1.11 |
| XVIII | 0.1% Tergitol TMN 100 | 0.72 |
| XVIII | 0.1% Tergitol TMN 100 0.1% Saponin | 0.99 |
| XVIII | 0.1% Tergitol TMN 100 0.2% Saponin | 0.88 |
| | | |
| XIX | 0% control | 0.32 |
| XIX | 0.1% NP40 (Igepal C-630A) | 1.00 |
| XIX | 0.1% Igepal C-630A 0.1% Saponin | 1.01 |
| XIX | 0.1% Igepal C-630A 0.2% Saponin | 0.89 |
| XIX | 0.1% Nonidet P40 Substitute (BioXtra) | 0.82 |
| XIX | 0.1% Nonidet P40 Substitute (BioXtra) 0.1% Saponin | 1.15 |
| XIX | 0.1% Nonidet P40 Substitute (BioXtra) 0.2% Saponin | 0.88 |
| XIX | 0.1% Triton X114 | 0.73 |
| XIX | 0.1% Triton X114 0.1% Saponin | 0.86 |
| XIX | 0.1% Triton X114 0.2% Saponin | 0.69 |
| | | |
| XXI | 0.1% Igepal CA-630 (NP40) | 1.00 |
| XXI | 0.1% Igepal CA-720 | 0.96 |
| XXI | 0.1% Igepal CA-720 + 0.1% Saponin | 1.79 |
| XXI | 0.1% Tergitol 15-S-7 | 1.07 |
| XXI | 0.1% Tergitol 15-S-7 + 0.1% Saponin | 1.37 |
| XXI | 0.1% Tergitol 15-S-9 | 1.11 |
| XXI | 0.1% Tergitol 15-S-9 + 0.1% Saponin | 1.26 |
| XXI | 0.1% Tergitol 15-S-30 | 1.10 |
| XXI | 0.1% Tergitol 15-S-30 + 0.1% Saponin | 1.28 |
| XXI | 0.1% Tergitol 15-S-40 | 1.04 |
| XXI | 0.1% Tergitol 15-S-40 + 0.1% Saponin | 1.09 |
| | | |
| XXII | 0.1% Igepal CA-630 (NP40) | 1.00 |
| XXII | 0.1% NP40 + 0.1% methyl-β-cyclodextrin | 0.92 |
| XXII | 0.1% NP40 + 0.1% heptakis(2,6-di-O-methyl)-β-cyclodextrin | 0.95 |
| XXII | 0.1% NP40 + 0.1% imipramine | 0.45 |
| XXII | 0.1% NP40 + 0,1% chlorpromazine | 0.57 |
| XXII | 0.1% NP40 + 0.1% saponin | 1.19 |
| XXII | 0.1% Igepal CO630 | 0.98 |
| XXII | 0.1% NP40 + 0.1% oleic acid | 0.21 |
| XXII | 0.1% NP40 + 0.1% cytochalasin B | 0.86 |
| XXII | 0.1% NP40 (3% EtOH) | 0.33 |
| XXII | 0.1% NP40 (3% DMSO) | 0.72 |
| | | |
| XXIV | 0 | 0.25 |
| XXIV | 0.1% Igepal CA-630 (NP40) | 1.00 |
| XXIV | 0.1% NP40 + 0.2% methyl-β-cyclodextrin | 0.90 |
| XXIV | 0.1% NP40 + 0.2% heptakis(2,6-di-O-methyl)-β-cyclodextrin | 1.04 |
| XXIV | 0.1% NP40 + 0.5% methyl-β-cyclodextrin | 0.45 |
| XXIV | 0.1% NP40 + 0.5% heptakis(2,6-di-O-methyl)-β-cyclodextrin | 0.39 |
| XXIV | 0.1% NP40 + 1% methyl-β-cyclodextrin | 0.46 |
| XXIV | 0.1% NP40 + 1% heptakis(2,6-di-O-methyl)-β-cyclodextrin | 0.50 |
| XXIV | 0.1% NP40 + 3% DMSO | 0.00 |
| XXIV | 0.1% NP40 + 3% DMF | 0.00 |
| | | |
| XXV | 0 | 0.23 |
| XXV | 0.1% Igepal CA-630 (NP40) | 1.00 |
| XXV | 0.1% NP40 + 0.1% cyclosporin A | 0.76 |
| XXV | 0.1% NP40 + 0.1% Filipin | 0.39 |
| XXV | 0.1% NP40 + 0.1% α-hederin | 0.00 |
| XXV | 0.1% NP40 + 0.1% hederagenin | 0.12 |
| XXV | 0.1% NP40 + 0.1% hederalchalcoside A | 0.00 |
| XXV | 0.1% NP40 + 0.1% sphingosine | 0.00 |
| XXV | 0.1% NP40 + 0.1% dioscin | 0.48 |
| XXV | 0.1% NP40 + 0.1% nystatin | 0.16 |
| XXV | 0.1% NP40 + 0.1% oleanolic acid | 0.15 |
| | | |
| | 0 | 0.37 |
| XXVI | 0.1% Igepal CA-630 (NP40) | 1.00 |
| XXVI | 0.1% NP40 + 0.01% cyclosporin A | 0.82 |
| XXVI | 0.1% NP40 + 0.01% Filipin (in DMF) | 0.30 |
| XXVI | 0.1% NP40 + 0.01% α-hederin | 0.48 |
| XXVI | 0.1% NP40 + 0.01% hederagenin | 0.47 |
| XXVI | 0.1% NP40 + 0.01% hederalchalcoside A | 0.52 |
| XXVI | 0.1% NP40 + 0.01% sphingosine | 0.57 |
| XXVI | 0.1% NP40 + 0.01% dioscin | 0.85 |
| XXVI | 0.1% NP40 + 0.01% nystatin (in DMF) | 0.21 |
| XXVI | 0.1% NP40 + 0.01% oleanolic acid (in DMF) | 0.18 |
| XXVI | 0.1% NP40 + 3% DMSO | 0.74 |
| XXVI | 0.1% NP40 + 3% DMF | 0.00 |

### Example 2: Evaluation of cell lysis efficiency and transcript capture in single-cells

This example illustrates certain techniques for barcoding nucleic acids using lysis reagents revealed in this invention. These examples are better understood together with Fig. 2-4. In an exemplary embodiment, the cells are introduced into a microfluidics device and are co-encapsulated along with hydrogel beads and assay reagents within microfluidic droplets. Once encapsulated the cells are lysed by the lysis reagents releasing the inner content such as mRNA. The hydrogel bead carries barcoding oligonucleotides covalently attached thereto. Once encapsulated the barcoding oligonucleotides are released by melting the hydrogel bead in the presence of chemical agent (e.g. reducing agent DTT). Alternatively, the barcoding oligonucleotides can be released by other means such as photo-illumination or enzymatic-cleavage. The nucleic acid molecules are attached to the barcoding oligonucleotide tags through the enzymatic (e.g. ligation, primer extension, PCR) or chemical (e.g. click-chemistry) reaction. It should be understood that certain applications may rely on nucleic molecule capture and barcoding without releasing barcoding oligonucleotides from the beads. For example, nucleic acid molecules of lysed cell could be captured on a bead within a droplet, as has been shown previously [39].

The hydrogel bead used in this example contained barcoding DNA sequence which schematically is indicated in Fig. 5.

As one non-limiting example, the fluids are delivered into the microfluidics device. The device has four inlets and one outlet. The inlets are used to introduce i) cells, ii) hydrogel beads, iii) lysis reagent and RT reaction mix and iv) carrier oil. Importantly, lysis reagents can be introduced along with hydrogel beads or along with RT reaction mix. The cells, hydrogel beads, and assay reagents can be introduced into a microfluidics chip through either inlet. Droplet generation occurs at or downstream the flow-focusing junction and co-encapsulated cells and hydrogel beads are then collected at the outlet. The flow rate of each inlet can be adjusted in order to obtain optimal flow conditions. The flow rates of all phases can be adjusted independently between 1 and 10,000 ul/h, depending on the particular application.

Once encapsulated, the nuclei acid molecules released from lysed cells can be processed further such as the mRNA molecules can be converted to cDNA using reverse transcription (RT). During the RT step the barcoding nucleotides anneal to poly(A) part of mRNA molecules via 3' end poly(T) tail, and get extended to cDNA.

In one particular example the reverse transcription (RT) reaction mix with and without collagenase was prepared as indicated in Table 6. 150µl of RT reaction mix was infused into a microfluidics chip along with SPMs and hydrogel beads in order to perform nucleic acid barcoding.

**Table 6. RT reaction mix composition.**

| **Reagent** | **Volume, µl** | **Concentration** |
|---|---|---|
| 5X RT buffer | 30 | 2X |
| dNTPs, 10mM each | 15 | 1 mM each |
| TSO primer, 0.5 mM 5'-AAGCAGTGGTATCAACGCAGAGTACATrGrGrG - 3' | 7.5 | 25 µM |
| Lysis reagents | 9 | 0.1-0.6% |
| Maxima H minus, 200 U/µL | 15 | 20 U/µl |
| Ribolock, 40 U/µL | 15 | 4 U/µl |
| Water | Up to 150 | |

Droplets carrying co-encapsulated cells, hydrogel beads and assay reagents may be collected off-chip and retain integrity. Droplet collected off-chip can be incubated at desirable temperature for an extended period of time. For example, collected droplets can be incubated at 50 ºC for 60 min followed by 15 min incubation at 85 ºC in order to perform RT reaction.

To release the barcoded nucleic acid (DNA or RNA) molecules, droplets can be broken by chemical or physical techniques. Typically, the emulsion droplets are broken using perfluoro-octanol. The water-in-oil droplets in this particular example were broken by adding 10% perfluoro-octanol (Sigma-Aldrich, 370533) onto the collected emulsion. The barcoded nucleic acid molecules present in aqueous phase of a broken emulsion can be amplified or further processed for sequencing. In one non-limiting example revealed here the aqueous phase of broken emulsion was spun down through Zymo Spin-IC column, the flow-through fraction was collected and purified 2-times with 0.8X AMPure magnetic beads (Beckman Coulter, A63880), and eluted in 20 µl of water. The barcoded-cDNA may be amplified by PCR. Tables 7 and 8 indicate the PCR reaction mix and cycling conditions for amplifying the barcoded cDNA.

**Table 7. cDNA amplification mix**

| Reagent | Volume, µl | Concentration in 2X mix |
|---|---|---|
| 2X KAPA HiFi ReadyMix | 25 | 1X |
| PCR primer mix*, 5 µM each | 5 | 0.5 µM each |
| Purified cDNA | 20 | |
| Total | 50 | |

| | | |
|---|---|---|
| * PCR primer mix | | |

| | |
|---|---|
| FWD primer | 5'-TACGGCGACCACCGAGATC-3' |
| REV primer | 5'-AAGCAGTGGTATCAACGCAGAG-3' |

**Table 8. PCR program settings**

| Step | Temperature | Time |
|---|---|---|
| Initial denaturation | 98°C | 00:03:00 |
| Denaturation | 98°C | 00:00:15 |
| Annealing | 67°C | 00:00:20 |
| Extension | 72°C | 00:01:00 |
| Go to step 2, 12 cycles (13 in total) | | |
| Final extension | 72°C | 00:01:00 |
| Hold | 4°C | Hold |

The amplified cDNA can be further processed to construct sequencing library. For example, the barcoded-cDNA amplified by PCR was purified 2-times with 0.6X AMPure magnetic beads, eluted into 20 µl of water and fragmented as follows (Table 9):

**Table 9. DNA fragmentation reaction composition**

| **Component** | **Volume, µL** |
|---|---|
| Amplified cDNA (6-9 ng/µL) | 5 |
| Water | 10.25 |
| NEBNext Ultra FS Reaction buffer (vortex before use) | 1.75 |
| NEBNext Ultra FS Enzyme mix (vortex before use) | 0.5 |
| Total: | 17.5 |

Samples were vortexed and spin-down and DNA fragmentation was carried out at 37°C for 8 min, followed by 30 min at 65°C. After reaction double size selection (0.6x-0.8X SPRI) was performed. Purified fragmented DNA was eluted in 17.5 µL of water and ligated to adapter using following reaction mix (**Table 10**):

**Table 10. Reaction composition for ligation of PCR adapter**

| **Component** | **Volume, µL** |
|---|---|
| Fragmented DNA | 17.5 |
| NEBNext Ultra II Ligation Master Mix | 7.5 |
| NEBNext Ultra Ligation Master Enhancer | 0.25 |
| Ligation adapter * | 1.25 |
| Water | 7.75 |
| Total: | 34.25 |

| | |
|---|---|
| (see next page) * Ligation adapter is a duplex DNA having two oligonucleotides: 5'-/5Phos/GATCGGAAGAGCACACGTCTGAACTCCAGTCAC/3ddC - 3' 5'- /5AmMC6/GCTCTTCCGATCT - 3' | |

Ligation was carried out at 20 °C for 15 min (lid at 30°C). After reaction total volume was brought to 100 µL, 0.8x ampure was performed and ligation product was eluted in 40 µL. Next indexing PCR was conducted by preparing the PCR reaction mix and cycling conditions indicated in Table 11 and 12 below:

**Table 11. PCR reaction composition**

| **Component** | **Volume**, **µL** |
|---|---|
| 2X KAPA HiFi ReadyMix | 25 |
| Template from previous step | 20 |
| Indexing primers* (5µM each) | 5 |
| Total: | 50 |

| | |
|---|---|
| Indexing primers P5 indexing primer: 5'-AATGATACGGCGACCACCGAGATCTACAC P7 index 6: 5'-CAAGCAGAAGACGGCATACGAGAT CAGTGG GTGACTGGAGTTCAGACGTG*T P7 index 7: 5'-CAAGCAGAAGACGGCATACGAGAT GTTGTC GTGACTGGAGTTCAGACGTG*T P7 index 8: 5'-CAAGCAGAAGACGGCATACGAGAT TGACAA GTGACTGGAGTTCAGACGTG*T | |

**Table 12. PCR cycling conditions**

| **Step** | **Temperature** | **Duration** | **Cycles** |
|---|---|---|---|
| Initial denaturation | 95°C | 3 min | 1 |
| Denaturation | 98°C | 20s | 11 |
| Annealing | 54°C | 30s | |
| Extension | 72°C | 20s | |
| Final extension | 72°C | 1 min | 1 |
| Hold | 4°C | Hold | |

The amplified DNA library was purified using double size selection (0.6-0.8X AMPure magnetic beads (Beckman Coulter, A63880)) and eluted in 15 µL. The DNA library quality was then verified on Agilent BioAnalyzer HS DNA chip (Agilent, 5067-4626). Once DNA library is generated the primary sequence of nucleic acids, including the barcode and unique molecular identifiers can be determined by sequencing. It should be understood that the above example can be used to prepare and analyse, as non-limiting examples, genomes, transcriptomes, epigenomes, single nucleotide polymorphisms, specific gene expression levels, non-coding RNA, entire genes or their sections, etc.

Sequencing of DNA libraries was performed on NextSeq Illumina instrument using NextSeq 2000 P3 reagents (50 Cycles) and following cycling numbers: Read 1 - 16 cycles, Read 2 - 58 cycles, i5 read - 8 cycles, i7 read - 6 cycles. The sequencing data was processed using STARsolo. According to the obtained metrics the single-cell transcriptomics libraries prepared using cholesterol-binding detergent or in combination with non-ionic detergents gave higher percentage of reads mapped to genes and higher fraction of reads mapped to cells. At similar sequencing depth, using saponin alone or in combination with non-ionic detergents generated higher UMI count and gene counts than regular conditions libraries (Fig. 3 and 4).

### Example 3: Evaluation of cell lysis efficiency and transcript capture of different types of cells

This is another example illustrating the advantage of the cell lysis reagents disclosed in this invention for obtaining improved barcoding of nucleic acids of single cells. In an exemplary embodiment, the 5 different types of cells [A549 (CCL-185), HeLa (CCL-2), MDA-MB-231 (HTB-26), PC3 (CRL-1435), K562 (CCL-243)] were mixed at equal proportion and a mixture of cells encapsulated in microfluidic droplets along with DNA barcoding hydrogel beads and assay/lysis reagents. In yet another exemplary embodiment, the 5 different types of cells, namely A375, Ker-Tomato, Caki-1, Jurkat, BxPC3 cells were mixed together at equal proportion and a mixture of cells encapsulated in microfluidic droplets along with DNA barcoding hydrogel beads and assay/lysis reagents. The lysis/RT conditions tested comprised different types of lysis agents that are listed in Table 13. The RT reaction mixture was prepared as described above (Table 6).

**Table 13. The list of lysis agents and their combinations.**

| Lysis agents | Reaction composition | Cell lines tested |
|---|---|---|
| IGEPAL | 0.3% Igepal CA630, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562, |
| | | A375, Ker-Tomato, Caki-1, Jurkat, BxPC3 |
| Triterpene glycoside | 0.3% Saponin, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562, |
| | | A375, Ker-Tomato, Caki-1, Jurkat, BxPC3 |
| TERGITOL | 0.3% Tergitol 15S9, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| BRIJ | 0.3% Brij58, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| TWEEN | 0.3% Tween20, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| n-dodecyl β-maltoside | 0.3% n-dodecyl β-maltoside, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| n-octyl β-glucopyranoside | 0.3% n-octyl β-glucopyranoside, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| Triterpene glycoside and IGEPAL mixture | 0.3% IgepalCA630, 0.3% Saponin, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562, |
| | | A375, Ker-Tomato, Caki-1, Jurkat, BxPC3 |
| Triterpene glycoside and TERGITOL mixture | 0.3% Tergitol 15S9, 0.3% Saponin, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| Triterpene glycoside and TWEEN mixture | 0.3% Tween20, 0.3% Saponin, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |
| Triterpene glycoside and BRIJ mixture | 0.3% Brij58, 0.3% Saponin, 1X RT buffer, 0.5 mM dNTP each, 25 µM TSO, 2 U/µL RNAse inhibitor (Ribolock), 10 U/µL RT enzyme (Maxima H-), 0.5X DPBS buffer, 10% Optiprep, 0.3% Pluronic F-127 | A549, HeLa, MDA-MB-231, PC3, K562 |

Following single cell isolation in water-in-oil droplets they were lysed by the lysis agent(s) supplemented in a reaction mixture to release the inner content such as mRNA. The barcoding oligonucleotides attached to hydrogel bead were released by means of photo-illumination. The hydrogel bead used in this example contained barcoding DNA sequence, which schematically is indicated in **Fig. 5****.** The mRNA molecules released from lysed cells were converted to cDNA using reverse transcription (RT) reaction. During the RT step the barcoding oligonucleotides annealed to poly(A) part of mRNA molecules via 3' end poly(T) tail, and were extended into cDNA by RT enzyme. 150 µl of RT reaction mix (Table 6) was infused into a microfluidics chip along with 150 µl cell suspension comprising K562, A549, HeLa, MDA-MB231 and PC3 cells, and DNA hydrogel beads. In yet another experiment, 150 µl cell suspension comprised a mixture of A375, Ker-Tomato, Caki-1, Jurkat, BxPC3 cells. The flow rates for injecting different fluids were: 250 µl/hr for RT reaction mix, 250 µl/hr for mixture of cells, 80 µl/hr for hydrogel beads, 800 µl/hr for the carrier oil supplemented with stabilizing surfactant. The volume of droplets was approximately 1 nl. Droplets collected off-chip were incubated at 42 ºC for 60 min to allow cDNA synthesis (nucleic acid barcoding) to occur and followed by 15 min incubation at 85 ºC.

To release the barcoded nucleic acid molecules, droplets were broken by adding 10% perfluoro-octanol onto the droplets. The aqueous phase of a broken emulsion was applied through Zymo Spin-IC column, the flow-through fraction was collected and purified 2-times with 0.8X AMPure magnetic beads (Beckman Coulter, A63880), and eluted in 20 µl of water. The barcoded-cDNA was then amplified by PCR. **Tables 7** and **8** indicate the PCR reaction composition and cycling conditions for amplifying the barcoded cDNA. The barcoded-cDNA amplified by PCR was purified 2-times with 0.6X AMPure magnetic beads, eluted into 20 µl of water and fragmented by assembling reaction indicated in **Table 9**. Fragmentation was carried out at 37 °C for 8 min, followed by 30 min at 65 °C. Next, fragmented DNA was purified with SPRI beads (e.g. using double size selection 0.6-0.8X). Purified fragmented-DNA was eluted in 17.5 µL of water and ligated to adapter by assembling a reaction mix indicated in Table 10. Ligation was carried out at 20 °C for 15 min, purified with 0.8X AMPure magnetic beads and eluted in 40 µL. Finally, indexing PCR was conducted by preparing the PCR reaction mix and cycling conditions indicated in Tables 11 and 12. The amplified DNA library was purified using double size selection (0.6-0.8X AMPure magnetic beads (Beckman Coulter, A63880)) and eluted in 15 µL. The DNA library quality was then verified on Agilent BioAnalyzer HS DNA chip (Agilent, 5067-4626) and sequenced on NextSeq Illumina instrument using NextSeq 2000 using following cycling numbers: Read 1 - 16 cycles, Read 2-58 cycles, i5 read - 8 cycles, i7 read - 6 cycles.

The sequencing data was processed using STARsolo. The solo-in-drops pipeline (https://github.com/jsimonas/solo-in-drops) was used to obtain prepare raw sequencing data for STARsolo to obtain cells x genes matrices and BAM files. For further analysis filtered matrices outputted by STARsolo default filtering parameters were used. Scanpy package and custom functions from Pfirschke et. al, 2022 (https://qithub.com/AllonMKlein/Pfirschke et al 2021) were used for filtering, normalization, selection of high variable genes using Fano factor, visualization with UMAP and clustering with spectral clustering. Doublets were identified using Scrublet package, batch correction were done using harmomy-py package. Differentially expressed gene analysis between different lysis conditions was done by performing two-sided Mann-Whitney U test and p-values were corrected with p-values correction with Benjamini-Hochberg false discovery rate method and family-wise error rate of 0.05. Significantly expressed genes were then sorted based on gene expression log2-fold change. Gene Set Enrichment Analysis was performed using gseapy package.

To generate sequencing saturation curves BAM files from STARsolo output were used to downsample the sequencing data to a series of different raw read number depth. The downsampling factors used were as follows - 0.05, 0.1, 0.2, 0.3, 0.4, 0.5, 0.6, 0.7, 0.8, 0.9, 1.0. Average UMI and gene counts per cell were generated at each sequencing depth and plotted against average reads per cell at different sequencing depths. Gene biotypes were extracted from GRCh38 reference genome gtf file.

The results presented in Fig. 6A and Fig. 6B show that triterpene glycoside (e.g. saponin) alone, or in combination with other lysis agents, is compatible with RT reaction and improves the transcript (UMI) detection in single-cells.

All publications, patents, and patent applications mentioned herein are hereby incorporated by reference in their entirety. In cases where the present specification and a document incorporated by reference include conflicting and/or inconsistent disclosure, the present specification shall control.

### References

1. Schuck, S., et al., Resistance of cell membranes to different detergents. Proceedings of the National Academy of Sciences of the United States of America, 2003. 100(10): p. 5795-5800.
2. le Maire, M., P. Champeil, and J.V. Moller, Interaction of membrane proteins and lipids with solubilizing detergents. Biochimica Et Biophysica Acta-Biomembranes, 2000. 1508(1-2): p. 86-111.
3. Vinardell, M.P. and M.R. Infante, The relationship between the chain length of non-ionic surfactants and their hemolytic action on human erythrocytes. Comparative Biochemistry and Physiology C-Pharmacology Toxicology & Endocrinology, 1999. 124(2): p. 117-120.
4. Vaidyanathan, S., B.G. Orr, and M.M.B. Holl, Detergent Induction of HEK 293A Cell Membrane Permeability Measured under Quiescent and Superfusion Conditions Using Whole Cell Patch Clamp. Journal of Physical Chemistry B, 2014. 118(8): p. 2112-2123.
5. Heerklotz, H. and J. Seelig, Correlation of membrane/water partition coefficients of detergents with the critical micelle concentration. Biophysical Journal, 2000. 78(5): p. 2435-2440.
6. Koley, D. and A.J. Bard, Triton X-100 concentration effects on membrane permeability of a single HeLa cell by scanning electrochemical microscopy (SECM). Proceedings of the National Academy of Sciences of the United States of America, 2010. 107(39): p. 16783-16787.
7. Groot, R.D. and K.L. Rabone, Mesoscopic simulation of cell membrane damage, morphology change and rupture by nonionic surfactants. Biophysical Journal, 2001. 81(2): p. 725-736.
8. Partearroyo, M.A., et al., Surfactant-Induced Cell Toxicity and Cell-Lysis - a Study Using B16 Melanoma-Cells. Biochemical Pharmacology, 1990. 40(6): p. 1323-1328.
9. Lingwood, D. and K. Simons, Detergent resistance as a tool in membrane research. Nature Protocols, 2007. 2(9): p. 2159-2165.
10. Melkonian, K.A., et al., Characterization of proteins in detergent-resistant membrane complexes from Madin-Darby canine kidney epithelial cells. Biochemistry, 1995. 34(49): p. 16161-16170.
11. Schroeder, R.J., et al., Cholesterol and sphingolipid enhance the Triton X-100 insolubility of glycosylphosphatidylinositol-anchored proteins by promoting the formation of detergent-insoluble ordered membrane domains. Journal of Biological Chemistry, 1998. 273(2): p. 1150-1157.
12. Lorent, J., et al., Induction of Highly Curved Structures in Relation to Membrane Permeabilization and Budding by the Triterpenoid Saponins, alpha- and delta-Hederin. Journal of Biological Chemistry, 2013. 288(20): p. 14000-14017.
13. Kilsdonk, E.P.C., et al., Cellular Cholesterol Efflux Mediated by Cyclodextrins. Journal of Biological Chemistry, 1995. 270(29): p. 17250-17256.
14. Nash, Z., et al., Protein characterization of triton insoluble detergent resistant membrane fractions in bovine rod outer segments. Investigative Ophthalmology & Visual Science, 2005. 46.
15. Schroeder, R., et al., Characterization of a Detergent-Resistant Lipid-Protein Complex Isolated from Epithelial-Cells That Is Enriched in Apical Membrane-Components. Molecular Biology of the Cell, 1992. 3: p. A306-A306.
16. Zhu, C., S. Preissl, and B. Ren, Single-cell multimodal omics: the power of many. Nat Methods, 2020. 17(1): p. 11-14.
17. Klein, A.M., et al., Droplet barcoding for single-cell transcriptomics applied to embryonic stem cells. Cell, 2015. 161(5): p. 1187-201.
18. Zheng, G.X., et al., Massively parallel digital transcriptional profiling of single cells. Nat Commun, 2017. 8: p. 14049.
19. Peterson, V.M., et al., Multiplexed quantification of proteins and transcripts in single cells. Nat Biotechnol, 2017. 35(10): p. 936-939.
20. Stoeckius, M., et al., Simultaneous epitope and transcriptome measurement in single cells. Nature Methods, 2017. 14(9): p. 865-+.
21. Lareau, C.A., et al., Droplet-based combinatorial indexing for massive-scale single-cell chromatin accessibility. Nat Biotechnol, 2019. 37(8): p. 916-924.
22. Clark, S.J., et al., scNMT-seq enables joint profiling of chromatin accessibility DNA methylation and transcription in single cells. Nat Commun, 2018. 9(1): p. 781.
23. Dey, S.S., et al., Integrated genome and transcriptome sequencing of the same cell. Nat Biotechnol, 2015. 33(3): p. 285-289.
24. Macaulay, I.C., et al., G&T-seq: parallel sequencing of single-cell genomes and transcriptomes. Nat Methods, 2015. 12(6): p. 519-22.
25. Angermueller, C., et al., Parallel single-cell sequencing links transcriptional and epigenetic heterogeneity. Nat Methods, 2016. 13(3): p. 229-232.
26. Hu, Y., et al., Simultaneous profiling of transcriptome and DNA methylome from a single cell. Genome Biol, 2016. 17: p. 88.
27. Hou, Y., et al., Single-cell triple omics sequencing reveals genetic, epigenetic, and transcriptomic heterogeneity in hepatocellular carcinomas. Cell Research, 2016. 26(3): p. 304-319.
28. Zheng, J., et al., A Modified SMART-Seq Method for Single-Cell Transcriptomic Analysis of Embryoid Body Differentiation. Methods Mol Biol, 2021.
29. Goetz, J.J. and J.M. Trimarchi, Transcriptome sequencing of single cells with Smart-Seq. Nat Biotechnol, 2012. 30(8): p. 763-5.
30. Hagemann-Jensen, M., et al., Single-cell RNA counting at allele and isoform resolution using Smart-seq3. Nat Biotechnol, 2020. 38(6): p. 708-714.
31. Weyant, R.S., P. Edmonds, and B. Swaminathan, Effect of ionic and nonionic detergents on the Taq polymerase. Biotechniques, 1990. 9(3): p. 308-9.
32. Koster, S., et al., Drop-based microfluidic devices for encapsulation of single cells. Lab on a Chip, 2008. 8(7): p. 1110-1115.
33. Clausell-Tormos, J., et al., Droplet-based microfluidic platforms for the encapsulation and screening of mammalian cells and multicellular organisms. Chem Biol, 2008. 15(5): p. 427-437.
34. Macosko, E.Z., et al., Highly Parallel Genome-wide Expression Profiling of Individual Cells Using Nanoliter Droplets. Cell, 2015. 161(5): p. 1202-14.
35. Abate, A.R., et al., Beating Poisson encapsulation statistics using close-packed ordering. Lab Chip, 2009. 9(18): p. 2628-31.
36. Zilionis, R., et al., Single-cell barcoding and sequencing using droplet microfluidics. Nature Protocols, 2017.12(1).
37. Christopher, G.F. and S.L. Anna, Microfluidic methods for generating continuous droplet streams. Journal of Physics D-Applied Physics, 2007. 40(19): p. R319-R336.
38. Xia, Y.N. and G.M. Whitesides, Soft lithography. Angew Chem Int Ed, 1998. 37(5): p. 551-575.
39. Macosko, E.Z., et al., Highly Parallel Genome-wide Expression Profiling of Individual Cells Using Nanoliter Droplets. Cell, 2015. 161(5): p. 1202-1214.

## Claims

1. A composition of cell lysis reagent comprising a first amphiphile that preferentially binds to fatty acids, phospholipids, glycolipids, and/or sterols, wherein the said triterpene glycoside amphiphile does not lower activity of the enzymatic reaction needed to synthesize DNA by more than 10%, and/or does not lower the activity of reverse transcriptase for the synthesis of copy DNA by more than 10%.

2. The composition of claim 1, wherein the first amphiphile is saponin.

3. The composition according to claim 1 or 2, further comprising a second amphiphile, wherein the second amphiphile is non-ionic and belongs to a class of detergents consisting of n-alkyl-glucopyranosides, n-alkyl-maltosides, n-alkyl-thioglucopyranosides, ethoxylated nonylphenols (known as different types of Tergitol), octylphenoxypolyethoxy ethanols (known as different types of Triton and Igepal), polyoxyethylene ethers (known as different types of Brij) and alkyl polyethylene glycol ethers, and genapols.

4. The composition according to any one of claims 1 to 2, wherein the first amphiphile belongs to a class of detergents that bind cholesterol, the class being one of CHAPSO/CHAPS, Digitonin, DC-cholesterol, Cholesteryl-PEG600, CHOBIMALT, CHAPSTEROL, Saponin, and cyclodextrin and the second amphiphile is one of, TERGITOL 15-S-7, TERGITOL 15-S-9, TERGITOL 15-S-30, TERGITOL 15-S-40, TERGITOL NP7, NP40, TRITON X100, GENAPOL C100, IGEPAL CA-630, IGEPAL CA-720, BRIJ58, BRIJ35, and TWEEN20.

5. The composition according to any one of claims 1 to 4, wherein the concentration of the first amphiphile or the first and the second amphiphile is in the range of 0.00001% to 10% (w/v), and preferably in the range of 0.001% to 1% (w/v).

6. A method of cell lysis and DNA synthesis, where the method comprising the steps:
adding cells, cell lysis reagent and DNA synthesis reagents to a reaction mixture,
lysing the cells to release the desired component from the cells, wherein the desired component is nucleic acid; and
reverse transcribing, synthesizing, replicating and/or amplifying the nucleic acid.

7. The method according to claim 6, wherein the cell lysis reagents comprising a first amphiphile and/or the second, non-ionic amphiphile, and where DNA synthesis reagents comprise reverse transcription (RT) reagents, and DNA synthesis reagents comprise PCR reagents.

8. The method according to claim 6, wherein the reaction mixture is loaded in a microreactor such as micro-well, nano-well, water-in-oil droplet, or any other compartment that has volume below 10 µl and above 1 pl.

9. The method according to claim 6 or 8, wherein cell lysis and DNA synthesis on single-cells comprising the steps:
(i) combining cells, DNA barcodes, cell lysis reagents comprising triterpene glycoside with or without a second, non-ionic amphiphile, and DNA synthesis reagents in microreactor;
(ii) lysing the cells to release the desired component from the cells, wherein the desired component is nucleic acid; and
(iii) reverse transcribing, synthesizing, replicating and/or amplifying the nucleic acid.

10. The method according to any one claim from 6 to 9, comprising:
encapsulating a plurality of cells and a plurality of DNA barcoding beads in a plurality of droplets together with the lysis reagents and reagents needed for reverse transcribing, synthesizing, replicating and/or amplifying the nucleic acid, wherein each bead of the plurality of beads comprises a molecular tag, and some droplets comprise cells and beads, and wherein:
(ii) the plurality of droplets comprises cell lysis reagents, DNA synthesis reagents, RT reaction reagents, and no more than one cell and no more than one bead, and
(iii) the molecular tag of the bead in one droplet is distinguishable from the molecular tags of the beads in the other droplets;
(iv) lysing the cells; and
(v) converting RNA into cDNA by reverse transcriptase using molecular tags at RT primers.

11. The method according to claim 10, wherein the molecular tag is attached to the nucleic acid released by lysed cell, wherein the molecular tag is a barcoding DNA oligonucleotide, which allows the nucleic acid in one droplet to be uniquely identified from among the nucleic acids of the other droplets.

12. The method according to claim 6, wherein nucleic acid amplification is chosen from one of: polymerase chain reaction (PCR), loop-mediated isothermal amplification (LAMP), isothermal amplification, linear amplification, reverse transcription, replication, polymerase chain reaction (PCR), quantitative PCR, real-time PCR, digital PCR, reverse transcriptase PCR (RT-PCR), multiplex RT-PCR, and any combination thereof.

13. The method according to claim 8, wherein nucleic acid amplification is chosen from one of: polymerase chain reaction (PCR), loop-mediated isothermal amplification (LAMP), isothermal amplification, linear amplification, reverse transcription, replication, polymerase chain reaction (PCR), quantitative PCR, real-time PCR, digital PCR, reverse transcriptase PCR (RT-PCR), multiplex RT-PCR, and any combination thereof.

14. A kit for cell lysis comprising the composition of any one of claims 1-4.

15. A kit for DNA synthesis reaction comprising the reaction composition of claim 6.
